# EUROPEAN PATENT APPLICATION

(11) **EP 4 035 666 A1**
(43) Date of publication of application: **03.08.2022**
(21) Application number: 21305123.8
(22) Date of filing: 29.01.2021
(51) Int. Cl.: A61K 31/265, A61K 31/4453, A61K 31/5375, A61P 35/00

(54) **AMINOTHIOLESTER COMPOUND OR DERIVATIVE THEREOF FOR USE AS AN IMMUNOMODULATORY AGENT**

(71) Applicant: Advanced Biodesign, 69800 Saint Priest (FR)
(72) Inventor: PEREZ, Mileidys, 69008 LYON (FR); MARTIN, Guillaume, 69008 LYON (FR)
(74) Representative: Lavoix

(57) **Abstract**

The present invention relates to aminotiolesters compounds for use for modulating the immune response in a subject. In particular, said compounds are comprised in a lipidic nanocapsule. The invention further relates to a pharmaceutical composition and to combination products comprising at least one aminothiolester compound and an immune checkpoint inhibitor, and their use for the treatment of cancer.

## Description

The present invention relates to aminothiolesters compounds for use for modulating the immune response in a subject. In particular, said compounds are comprised in a lipidic nanocapsule. The invention further relates to a pharmaceutical composition and to combination products comprising at least one aminothiolester compound and an immune checkpoint inhibitor, and their use for the treatment of cancer.

### Background of the invention

The modulation of the immune response is an important treatment axis for many diseases. Indeed, it is for example important to improve the immune response in patients suffering from infections.

The stimulation of the immune response is moreover an important mechanism in many respects.

Neutrophils have long been considered as an integral part of the innate immune response. Indeed, neutrophils have historically been characterized as first responder cells vital to host survival due to their ability to contain and eliminate bacterial, parasites, viral and fungal pathogens (Witter AR, Okunnu BM, Berg RE. The Essential Role of Neutrophils during Infection with the Intracellular Bacterial Pathogen Listeria monocytogenes. J Immunol. 2016; 197(5):1557-1565). Under infectious conditions, granulopoiesis increases and neutrophils are recruited into circulation and then to peripheral sites of infection.

Specific adhesion molecules expressed on neutrophils include LFA-1 (CD11 a/CD18) and Mac-1 (CD11 b/CD18, CR3). LFA-1 expression restricts neutrophil recruitment to sites of infection leading to increased bacterial burden, while Mac-1, particularly the CD11b component, is essential for neutrophil recruitment and control of infection (Witter AR, Okunnu BM, Berg RE. The Essential Role of Neutrophils during Infection with the Intracellular Bacterial Pathogen Listeria monocytogenes. J Immunol. 2016; 197(5):1557-1565).

The inventors of the present invention have surprisingly found that aminothiolesters compounds of formula (I) described below are able to stimulate and promote maturation and activation of neutrophils and to treat and/or prevent infectious pathologies

Furthermore, macrophages are considered frontier soldiers of innate immunity. Due to their immune surveillance roles, macrophages sense a wide spectrum of stimuli, spanning from viral, microbial and parasite antigens, immune complexes, and apoptotic or necrotic cells to various mediators released by other cells (Röszer T. Understanding the Mysterious M2 Macrophage through Activation Markers and Effector Mechanisms. Mediators Inflamm. 2015;2015:816460). In response to the stimulus they sense, macrophages are activated, which allows them to combat the pathogens, exert an immunomodulatory role, and maintain tissue integrity (F.O.Martinezand S. Gordon,"The M1 and M2 paradigm of macrophage activation: time for reassessment," F1000Prime Reports,vol.6,article 13,2014). In the recent decade, a model has been developed which describes the complex mechanism of macrophage activation as a polarization towards two opposite states, the M1 or classical, and the M2 or alternative activation (F.O.Martinezand S. Gordon,"The M1 and M2 paradigm of macrophage activation: time for reassessment," F1000Prime Reports,vol.6,article 13,2014).

The M2 macrophages have high phagocytosis capacity, producing extracellular matrix (ECM) components, angiogenic and chemotactic factors, and IL-10 (L. Fuentes, T. Roszer, and M. Ricote, "Inflammatory mediators and insulin resistance in obesity: role of nuclear receptor signaling in macrophages," Mediators of Inflammation,vol.2010, Article ID 219583, 10 pages, 2010). In addition to the pathogen defense, M2 macrophages clear apoptotic cells, can mitigate inflammatory response, and promote wound healing ( A. Sica and A. Mantovani, "Macrophage plasticity and polarization: in vivo veritas," The Journal ofClinical Investigation,vol. 122, no. 3, pp. 787-795, 2012). Not surprisingly, they are widely termed in the current literature as anti-inflammatory, pro-resolving, wound healing, tissue repair, and trophic or regulatory macrophages and considered as benign opposites of the M1 activated macrophages (J. W. Pollard, "Trophic macrophages in development and disease," Nature Reviews Immunology,vol.9,no. 4, pp.259-270, 2009). Also, M2 macrophages have complex roles outside the context of inflammation, such as organ morphogenesis, tissue turnover, and endocrine signaling (J. W. Pollard, "Trophic macrophages in development and disease," Nature Reviews Immunology,vol.9,no. 4, pp.259-270, 2009; F.O.Martinez and S. Gordon,"The M1 and M2 paradigm of macrophage activation: time for reassessment," F1000Prime Reports,vol.6,article 13,2014; S. J. Forbes and N. Rosenthal, "Preparing the ground for tissue regeneration: from mechanism to therapy," Nature Medicine, vol. 20, pp. 857-869, 2014.; E. Muraille, O. Leo, and M. Moser, "TH1/TH2 paradigm extended: macrophage polarization as an unappreciated pathogen-driven escape mechanism?" Frontiers in Immunology, vol.5, article 603, 2014). These M2 macrophage tasks have biomedical impact. For instance, sustaining the M2-like state of some tissue resident macrophages, such as Kupffer cells and adipose tissue macrophages, would diminish the production of inflammatory mediators and thus may be a therapeutic approach to treat autoimmune disorders such as rheumatoid arthritis, inflammatory bowel disease, systemic lupus erythematosus, multiple sclerosis, psoriasis and metabolic inflammatory diseases such as atherosclerosis, type 2 diabetes, chronic kidney disease, non-alcoholic fatty liver disease, and obesity, including visceral obesity that affects important metabolic tissues such as adipose tissue, liver, skeletal muscle, pancreas, intestines, and hypothalamus, among others. Finally, because M2 macrophages are also indispensable players in organ development, tissue turnover, and angiogenesis, the tissue remodeling activities of the M2 macrophages have potential use in regenerative medicine.
In the context of tissue repair and regeneration, uncontrolled amplitude and duration of inflammation prevents regeneration, for instance, chronic inflammatory environment, such as in rheumatoid arthritis, impede regeneration. TRAF6 is considered to be a central factor that trigger and propagates type 17 immune responses in host defence and inflammatory diseases, including autoimmune disorders like in the case of rheumatoid arthritis (Teruki Dainichi, Reiko Matsumoto, Alshimaa Mostafa, Kenji Kabashima, "Immune Control by TRAF6-Mediated Pathways of Epithelial Cells in the EIME (Epithelial Immune Microenvironment)", Front Immunol, 16;10:1107, 2019). TRAF6 is also a pivotal mediator of NF-kB pathway, which is in turn important for the induction of genes associated with immune and inflammatory responses and oncogenesis.

The inventors of the present invention have surprisingly found that aminothiolesters compounds of formula (I) described below are able to induce mobilization of monocyte-associated macrophages and activate M2-type macrophage.

In addition, an emerging area for cancer therapy has been focused on the use of immune checkpoint inhibitors (ICI) targeting cytotoxic T-lymphocyte-associated protein 4 (CTLA-4), programmed cell death 1 (PD-1) receptor and their ligands (PD-L1 and PD-L2), among others, to boost immune system for the destruction of tumors. (Gilad, Y., Eliaz, Y., Yu, Y. et al. Author Correction: Drug-induced PD-L1 expression and cell stress response in breast cancer cells can be balanced by drug combination. Sci Rep. 2020; 10, 4463). Checkpoint blockade is a systemic approach which mostly targets the patient's immune system, rather than the cancer cells themselves, in order to unleash the immune response and reactivate exhausted T cells to act against cancer cells. Of note are the encouraging survival rates and durability of response in tough-to-cure types of cancers such as in advanced metastatic melanomas. However, 30%-60% of patients show no response to ICI in spite of the often-significant interaction between tumours and the immune system. Thus, a strong impetus exists to improve sensitivity to immunotherapies, possibly by combining it with other drugs.

Because the administration of ICPIs boosts the immune system, the main safety concern of ICI use is the induction of immune-related adverse events (irAEs) (Ref). Rash, psoriasis, endocrinopathies, hepatitis, colitis, pneumonitis, arthritis, and myositis have been described in ICI-treated individuals, and the spectrum of irAEs is still expanding.

An additional benefit in the context of cancer immunotherapy could be the attenuation of immune-related adverse events (irAEs) helping to manage immune tolerance and avoid the emergence of auto-immunity.

The inventors of the present invention have surprisingly found that aminothiolesters compounds of formula (I) described below are able to improve tumor sensitivity to immunotherapies and enhance antitumoral responses at the tumor site while attenuating the occurrence of irAEs.

### Summary of the invention

The inventors of the present invention have shown that the aminothiolesters of formula (I) described below have immunomodulating properties.

They have further shown that they can be used in combination with ICI, in particular for the treatment of cancer. Surprisingly, they have showed that this combination is synergistic in the prevention and/or treatment of cancer.

As such, the present invention relates to at least one compound of formula (I): 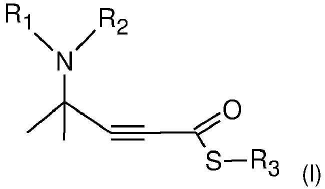 wherein:
- R₁ and R₂, identical or different, are chosen among a C₁-C₁₀ alkyl group, a phenyl, a benzyl, CHR₅CHR₆OR₄ and (CHR₅)ᵥOR₄, or R₁ and R₂ together with the nitrogen atom to which they are linked form an heterocycle, in particular a piperidine or a morpholine;
said phenyl and benzyl being optionally substituted by one or more substituents chosen from: linear or branched (C₁-C₇)alkyl, halogen, NO₂ and CONH₂ ;
- R₃ is chosen from linear or branched (C₁-C₇)alkyl,
- R₄ is chosen from: H, linear or branched (C₂-C₇)alkyl, linear or branched (C₂-C7)alkenyl, -CONR₇R₈, aryl, heteroaryl, (C₂-C₇)cycloalkyl, linear or branched -(C₁-C₇)alkyl-aryl and linear or branched -(C₁-C₇)alkyl-heteroaryl;
said aryl, (C₂-C₇)cycloalkyl, and heteroaryl being optionally substituted by one or more substituents chosen from: halogen, linear or branched (C₁-C₇)alkyl optionally substituted by one or more halogen atom, linear or branched (C₁-C₇)alkoxy optionally substituted by one or more halogen atom, -COOH, aryl, -NRR', -NO₂, or said aryl and heteroaryl being optionally fused to form an heterocycloalkyl;
- R₅ and R₆ identical or different are independently chosen from:
   - H and linear or branched (C₁-C₇)alkyl, or
   - R₅ and R₆ are linked together to form with the carbon atoms to which they are attached a cycloalkyl, aryl or heteroaryl, or
   - R₅ is H and R₁ and R₆ are linked together to form with the nitrogen atom linked to R₁ an heterocycloalkyl or heteroaryl, or
   - R₆ is H and R₁ and R₅ are linked together to R₁ to form with the nitrogen atom linked to R₁ an heterocycloalkyl;
- v is chosen from 2 to 4;
- R₇ is -(C₁-C₃)alkyl;
- R₈ is -(C₁-C₃)alkylNRR';
- R and R' identical or different, are independently chosen from H and linear or branched (C₁-C₇)alkyl,
or its pharmaceutically acceptable salts or optical isomers, racemates, diastereoisomers, enantiomers or tautomers;
for use for modulating the immune response in a subject.

In particular, said at least one compound of formula (I) is comprised in a lipidic nanocapsule.

The invention also relates to a pharmaceutical composition comprising at least one compound of formula (I) as defined herein, and an immune checkpoint inhibitor (ICI).

It further relates to a pharmaceutical composition comprising at least one compound of formula (I) as defined herein, and an immune checkpoint inhibitor (ICI), for use in the prevention and/or treatment of cancer.

### Detailed description of the invention

### Compounds of formula (I)

By "at least one compound of formula I", is meant 1, 2, 3, 4, 5, 6, 10, 15 or more compounds of formula (I).

When more than one compound of formula (I) is contemplated, this compound can be the same or different compounds of formula (I), in particular the same.

The compounds of formula (I) are as mentioned above and are described in the patent EP1296946 and in the patent application PCT/EP2020/071640, as well as their process of preparation.

In particular, the compounds of formula (I) are characterized in that R₃ is linear or branched (C₁-C₇)alkyl, preferably methyl, R₁ is linear or branched (C₁-C₇)alkyl, preferably methyl, R₂ is a C₁-C₁₀ alkyl group, preferably a methyl or octyl, CHR₅CHR₆OR₄, (CHR₅)ᵥOR₄ or R₁ and R₂ together with the nitrogen atom to which they are linked form an heterocycle, in particular a morpholine, and R₅ and R₆ are H.

For example, R₄ is chosen from H, linear or branched (C₂-C₇)alkyl, linear or branched (C₂-C₇)alkenyl, -CONR₇R₈, (C₂-C₇)cycloalkyl, linear or branched -(C₁-C₇) alkyl-heteroaryl, aryl or benzyl; said (C₂-C₇)cycloalkyl being substituted by one or more substituents chosen from: linear or branched (C₁-C₇)alkyl; said benzyl being optionally substituted by one or more substituents chosen from: linear or branched (C₁-C₇)alkyl optionally substituted by one or more halogen atoms, linear or branched (C₁-C₇)alkoxy optionally substituted by one or more halogen, halogen, or said benzyl optionally being fused to form 1,3-benzodioxole.

Alternatively, in particular, R₄ is chosen from H, linear or branched (C₂-C₇)alkyl, linear or branched (C₂-C₇)alkenyl, -(C₁-C₇)alkyl-heteroaryl, aryl, -(C₁-C₇)alkyl-aryl or benzyl; said benzyl being optionally substituted by one or more substituents chosen from: linear or branched (C₁-C₇)alkyl optionally substituted by one or more halogen atoms, linear or branched (C₁-C₇)alkoxy optionally substituted by one or more halogen atoms, halogen or pyridyl, or said benzyl optionally being fused to form 1,3-benzodioxole.

Alternatively, in particular, R₅ and R₆ are H and R₄ is chosen from H, linear or branched (C₂-C₇)alkyl, linear or branched (C₂-C₇)alkenyl, linear or branched -(C₁-C₇)alkyl-heteroaryl, -(C₁-C₇)alkyl-aryl or benzyl linear or branched; said benzyl being optionally substituted by one or more substituents chosen from: linear or branched (C₁-C₇)alkyl optionally substituted by one or more halogen atoms, linear or branched (C₁-C₇)alkoxy optionally substituted by one or more halogen atoms, halogen.

Alternatively, more particularly, the compounds of formula (I) are characterized in that R₅ and R₆ are H and R₄ is chosen from (C₂-C₇) cycloalkyl, linear or branched -(C₁-C₇)alkyl-heteroaryl, or benzyl; preferably benzyl; said (C₂-C₇) cycloalkyl being substituted by one or more substituents chosen from: linear or branched (C₁-C₇)alkyl, said benzyl being optionally substituted by one or more substituents chosen from: linear or branched (C₁-C₇)alkyl optionally substituted by one or more halogen atom, linear or branched (C₁-C₇)alkoxy optionally substituted by one or more halogen atom, halogen.

As a variant, even more particularly, R₁ is methyl and R₄ is chosen from: H, CONR₇R₈ with R₇ being methyl and R₈ being NRR' with R and R' being methyl, ethyl, propene, benzyl, pyridyl, benzyloxybutyl, methyl-cyclohexenyl substituted by one or more methyl, and benzyl substituted by one or more fluorine, chlorine, methoxy or methyl.

As a variant, even more particularly, R₁ is a methyl and R₄ is chosen from: H, ethyl, propene, benzyl, pyridyl, benzyloxybutyl and benzyl substituted by one of several fluorine, chlorine, methoxy or methyl.
Even more particularly, said at least one compound of formula (I) is chosen from:
- S-methyl 4-[2-ethoxyethyl(methyl)amino]-4-methyl-pent-2-ynethioate;
- S-methyl 4-[2-allyloxyethyl(methyl)amino]-4-methyl-pent-2-ynethioate;
- S-methyl 4-[2-benzyloxyethyl(methyl)amino]-4-methyl-pent-2-ynethioate;
- S-methyl 4-methyl-4-[methyl-[2-(m-tolylmethoxy)ethyl]amino]pent-2-ynethioate;
- S-methyl 4-[2-[(3,4-dimethylphenyl)methoxy]ethyl-methyl-amino]-4-methyl-pent-2-yneth ioate;
- S-methyl 4-[2-[(4-methoxyphenyl)methoxy]ethyl-methyl-amino]-4-methyl-pent-2-yneth ioate;
- S-methyl 4-[2-[(3,4-dimethoxyphenyl)methoxy]ethyl-methyl-amino]-4-methyl-pent-2-yneth ioate;
- S-methyl 4-[2-[(3-chlorophenyl)methoxy]ethyl-methyl-amino]-4-methyl-pent-2-ynethioate;
- S-methyl 4-[2-[(3-fluorophenyl)methoxy]ethyl-methyl-amino]-4-methyl-pent-2-ynethioate;
- S-methyl 4-methyl-4-[methyl-[2-(2-pyridylmethoxy)ethyl]amino]pent-2-ynethioate;
- S-methyl 4-methyl-4-[methyl-[2-(3-pyridylmethoxy)ethyl]amino]pent-2-ynethioate;
- S-methyl 4-methyl-4-[methyl-[2-(4-pyridylmethoxy)ethyl]amino]pent-2-ynethioate;
- S-methyl 4-((4-(benzyloxy)butyl)(methyl)amino)-4-methylpent-2-ynethioate;
- S-methyl 4-((2-hydroxyethyl)(methyl)amino)-4-methylpent-2-ynethioate;
- S-methyl 4-methyl-4-[methyl-[2-(2-naphthylmethoxy)ethyl]amino]pent-2-ynethioate;
- S-methyl 4-methyl-4-[methyl-[2-[(2,6,6-trimethylcyclohexen-1-yl)methoxy]ethyl]amino ]pent-2-yneth ioate;
- S-methyl 2,5,10,11,11-pentamethyl-6-oxo-7-oxa-2,5,10-triazatetradec-12-yne-14-thioate;
- S-methyl 4-methyl-4-[methyl(2-phenoxycyclopentyl)amino]pent-2-ynethioate;
- (S)-S-methyl 4-(2-((benzyloxy)methyl)pyrrolidin-1-yl)-4-methylpent-2-ynethioate;
- S-methyl 4-[3(benzyloxy)-1pyrrolidinyl])-4-methylpent-2-ynethioate;
- S-methyl 1-4-Dimethylamino-4-methyl-pent-2-ynethioate ;
- S-methyl 5- 4-Methyl-4-morpholin-4-yl-pent-2-ynethioate;
- S-methyl 4-methyl-4- [methyl (octyl) amino] pent-2-ynethioate;
or its pharmaceutically acceptable salts or optical isomers, racemates, diastereoisomers, enantiomers or tautomers.

Unless specified otherwise, the terms used hereabove or hereafter as regards to the compounds of formula (I) have the meaning ascribed to them below:
- v is chosen from 2 to 4 means that the substituent "CHR₅" is repeated twice CHR₅CHR₅OR₄, three times CHR₅CHR₅CHR₅OR₄ or four times CHR₅CHR₅CHR₅CHR₅OR₄.
- "halogen" refers to fluorine, chlorine, brome or iodine atom, in particular fluorine or chlorine atom.
- "alkyl" represents an aliphatic-hydrocarbon group which may be straight or branched, having 1 to 10, 1 to 7 or 2 to 7 carbon atoms in the chain (C₁-C₁₀) alkyl, (C₁-C₇)alkyl or (C₂-C₇)alkyl, unless specified otherwise. In particular, alkyl groups have 1 to 3 carbon atoms in the chain (C₁-C₃)alkyl. Branched means that one or more alkyl groups such as methyl, ethyl or propyl are attached to a linear alkyl chain. Exemplary alkyl groups include methyl, ethyl, n-propyl, i-propyl, n-butyl, t-butyl, octyl, 2,2-dimethylbutyl, n-pentyl, n-hexyl, n-heptyl, in particular methyl, ethyl or octyl.
- "alkenyl" refers to an aliphatic hydrocarbon group containing a carbon-carbon double bond and which may be straight or branched having 2 to 7 carbon atoms in the chain (C₂-C₇)alkenyl, unless specified otherwise. Preferred alkenyl groups have 2 to 3 carbon atoms in the chain (C₂-C₃)alkenyl. Exemplary alkenyl groups include ethenyl, n-propenyl, i-propenyl, n butenyl, i-butenyl, 2,2-dimethylbut-1-enyl, n-pentenyl, in particular propenyl.
- "alkoxy" represent an alkyl group as previously defined singular bonded to oxygen. Examples of linear or branched (C₁-C₇)alkoxy include methoxy (CH₃O-) and ethoxy (CH₃CH₂O-) .
- "aryl" refers to an aromatic monocyclic or multicyclic hydrocarbon ring system of 6 to 14 carbon atoms, preferably of 6 to 10 carbon atoms. Exemplary aryl groups include phenyl, naphthyl, benzyl, phenanthryl, biphenyl, in particular phenyl.
- "heteroaryl" refers to a 5 to 14, preferably 5 to 10 membered aromatic mono-, bior multicyclic ring wherein at least one member of the ring is a hetero atom. Hetero atoms can be O or N, in particular N. In particular, each ring comprises from 1 to 3 hetero atoms. Examples include pyrrolyl, pyridyl, piperidinyl, pyrazolyl, pyrimidinyl, pyrazinyl, indolyl, imidazolyl, in particular pyridyl.
- "cycloalkyl" refers to a saturated monocyclic or bicyclic non-aromatic hydrocarbon ring of 2 to 7 carbon atoms, preferably 3 to 6 carbon atoms, which can comprise one or more unsaturation. Specific examples of monocyclic cycloalkyl groups include cyclopropyl, cyclobutyl, cyclopentyl, cyclopentenyl, cyclohexyl, cyclohexenyl. Preferably, the cycloalkyl group is cyclohexenyl.
- "-(C₁-C₇)alkyl-aryl" or "-(C₁-C₇)alkyl-heteroaryl" means that R₄ is linked to the oxygen atom by the carbon of the alkyl group; in particular -(C₁-C₇)alkyl-aryl is a benzyl.
- -"heterocycle" or "heterocyclloalkyl" refers to a saturated or partially unsaturated non aromatic stable 3 to 14, preferably 5 to 10-membered mono, bi or multicyclic rings which can optionally be bridged and wherein at least one member of the ring is a hetero atom. Typically, heteroatoms include, but are not limited to O or N. In particular, each ring comprises from 1 to 3 hetero atoms. Suitable heterocycles are also disclosed in the Handbook of Chemistry and Physics, 76th Edition, CRC Press, Inc., 1995-1996, pages 225 to 226, the disclosure of which is hereby incorporated by reference. Examples of heterocycloalkyl include, but are not limited to tetrahydropyridyl, tetrahydropyranyl, pyrrolidinyl, piperidyl, morpholinyl, imidazolidinyl, or benzodioxole, in particular 1,3 benzodioxole.
- The term "substituted" refers to, unless specified otherwise, a substitution with one or more substituents, which may be identical or different, for example chosen from linear or branched (C₁-C₇)alkyl, halogen, NO₂ and CONH₂, linear or branched (C₁-C₇)alkyl substituted by one or more halogen atom, linear or branched (C₁-C₇)alkoxy, linear or branched (C₁-C₇)alkoxy substituted by one or more halogen atom, aryl, -COOH, -COOCH₂CH₃, -NRR', NH₂, NHalkyl and N(alkyl)₂. Examples include in particular methyl, methoxy, chlorine, fluorine, CF₃ and OCF₃.

The compounds of formula (I) as described herein can comprise one or more asymmetric carbon atoms. They can therefore exist in the form of enantiomers or diastereoisomers. These enantiomers and diastereoisomers, as well as their mixtures, including racemic mixtures, form part of the invention.

The compounds of formula (I) as described herein can be provided in the form of a free base or in the form of addition salts with acids, which also form part of the invention.

These salts are advantageously prepared with pharmaceutically acceptable acids, but salts with other acids, useful for example for the purification or for the isolation of the compounds of formula (I) as described herein, also form part of the invention.

As used herein, the expression "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like, including mono, di or tri-salts thereof; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 20th ed., Mack Publishing Company, Easton, PA, 2000, the disclosure of which is hereby incorporated by reference.

### Process of preparation of compounds of formula (I)

The compounds of formula (I) may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, the compounds of formula (I) can be prepared according to the processes described in patent EP1296946 and patent application PCT/EP2020/071640.

It will be appreciated that the compounds of formula (I) may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of formula (I) may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxyl, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 4th ed.(2007), John Wiley & Sons Inc., 1999; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule, and unless otherwise indicated. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, it is found convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

For example, a compound of formula (I) can be obtained by :
a) reacting a compound of formula (II) with an organic or inorganic acid 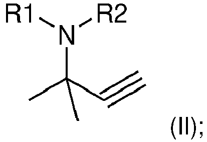
b) reacting the compound obtained in step a) with a base ;
c) reacting the compound obtained in step b) with CO₂ ;
d) reacting the compound obtained in step c) with alkyl chloroformate, a reagent able of forming, with the compound obtained in step c), an acid halide or a reagent able of forming, with the compound obtained in step c), a mixed anhydride ;
e) reacting the compound obtained in step d) with an anion precursor compound SMe-;
wherein R1 and R2 are as defined herein.

In particular, the base of step b) has a pKa greater than 25, preferably the base used in step b) is selected from lithium or magnesium bases, preferably the base is selected from butyllithium, or hexyllithium.

In particular, the compound of formula (II) is obtained by a step a1) of reaction between 3-chloro-3-methylbut-1-yne with R1R2NH in an aqueous medium.

In particular, said compound obtained in step a1) is purified by one or more filtrations, for example in filtration or in a succession of 2 to 10 filtrations, preferably in a succession of 2 to 5 filtrations, for example in 4 filtrations.

In one embodiment, 3-chloro-3-methylbut-1-yne is obtained by a reaction step of reacting 2-methylbut-3-yn-2-ol with hydrochloric acid in the presence of a copper catalyst.

In another embodiment, the acid is an inorganic acid chosen from hydrochloric acid, phosphoric acid, nitric acid, sulfuric acid, preferably hydrochloric acid.

In another embodiment, step d) is carried out with:
- an alkyl chloroformate having a (C₁-C₆)alkyl, which may comprise at least one double bond, preferably methyl, ethyl, isoprenyl, tert-butyl or isobutyl chloroformate, preferably isobutyl chloroformate; or
- a reagent capable of forming with the compound obtained in step c) a mixed anhydride chosen from acid chlorides, for example pivaloyl chloride; or
- a reagent capable of forming, with the compound obtained in step c), an acid halide chosen from SOCl₂, COCl₂, PCl₃, PCl₅, PBr₃ or PPh₃Br₂.

In one embodiment, the anion precursor compounds SMe- are chosen from the salts of formula XSMe in which X represents an alkali metal or alkaline earth metal, for example Na, methyl mercaptan, or (SMe)₂, preferably NaSMe.

This process is described in detail in the patent application PCT/EP2017/053457, from which the content is incorporated by reference.

Alternatively, a compound of formula (I) can be prepared from the corresponding acetylenic amine treated successively by BuLi, COS and Mel. A detailed process of preparation can be found for example in G.Quash et al., European Journal of Medicinal Chemistry 43 (2008) 906-916, from which the content is incorporated by reference, in particular in the part 2 of the Material and Methods section.

The above reactions can be carried out by the skilled person by applying or adapting the methods illustrated in the examples hereinafter.

Further, the process may also comprise the additional step of isolating the compound of formula (I) or (II). This can be done by the skilled person by any of the known conventional means, such as the recovery methods described above.

Generally, the starting products are commercially available mainly from Aldrich or Acros or other typical chemicals supplier or may be obtained by applying or adapting any known methods or those described in the examples.

### Lipidic nanocapsule

As mentioned above, in one embodiment, the at least one compound of formula (I) is comprised in a lipidic nanocapsule.

By "lipidic nanocapsule" or NCL, is meant a solvent-free formulation having an oil as solubilizing agent making it possible to encapsulate liposoluble active ingredients.

In the context of the present invention and as mentioned herein, the nanocapsule comprises an oily core in particular liquid/semi-liquid at room temperature and a shell surrounding the oily core in particular rigid at room temperature and whose melting/ transition temperature is high (i.e. in particular between 40°C and 85°C).). The core of the particle is therefore made up of oil, here medium chain triglycerides. The active principle (at least one compound of formula (I)) is solubilized in this phase in the center of the nanocapsule. The surface of the nanocapsule or shell is formed from hydrophilic and lipidic surfactants.

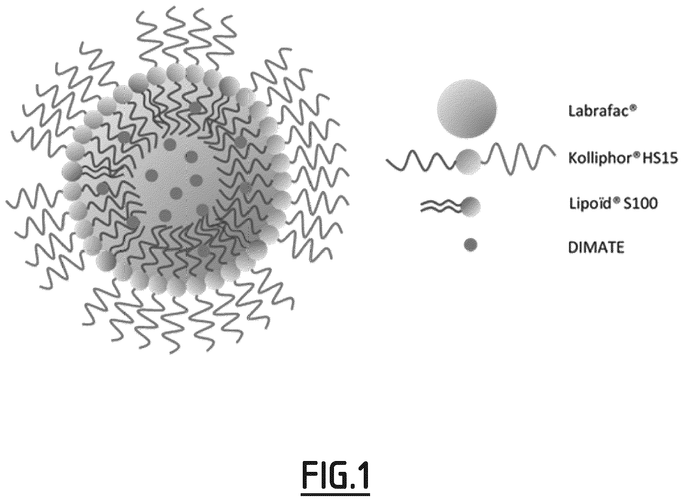

This structure can be illustrated by Figure 1.

In particular, said nanocapsule comprises:
- an oily core comprising between 25 and 90% by weight of medium chain triglycerides, preferably between 60 and 80%, relative to the total weight of the nanocapsule, and said at least one compound of formula (I); and
- a shell surrounding the oily core, comprising between 3 and 25% by weight relative to the total weight of the nanocapsule of at least one lipid surfactant, and at least one hydrophilic surfactant; and
   - in which the ratio by weight relative to the total weight of the nanocapsule between the medium chain triglycerides and said at least one compound of formula (I) is of at least 4.

In particular, said nanocapsule has a diameter of between 25 and 115 nm, more particularly between 40 and 80 nm, and for example of 30, 40, 45, 50, 55, 60, 65 or 70 nm.

This size makes it possible in particular to ensure optimum efficiency of the nanocapsule *in vivo.*

In particular, said nanocapsule has a PDI or polydispersity index below 0.2, in particular below 0.1. This index, which is also called dispersity, has the common sense known to those skilled in the art, i.e. the measure of the heterogeneity of the sizes of molecules or particles in a mixture. This allows in particular that the size distribution of the nanocapsules is monodisperse.

As mentioned above, the oily core comprises between 25 and 90% by weight of medium chain triglycerides, preferably between 60 and 80%, relative to the total weight of the nanocapsule, and for example, 40, 50, 55, 60 or 70 % by weight of medium chain triglycerides.

By "medium chain triglycerides" is meant the common sense known to those skilled in the art, i.e. triglycerides in which the three hydroxyl groups of glycerol are esterified with medium chain fatty acids.

The term "medium chain" is understood to mean in particular chains of 6 to 12 carbon atoms.

They are notably present in coconut oil, palm oil and butter.

In particular, the medium chain triglycerides are chosen from mixtures of triglycerides of saturated fatty acids, in particular of caprylic acid (octanoic) and of capric acid (decanoic).

The fatty acids can be obtained from the oil extracted from the hard, dried fraction of the albumen of *Cocos nucifera L.* or from the oil extracted from the dried albumen of *Elaeis guineensis Jacq.* meeting the prerequisites of the European Pharmacopoeia such as, more particularly Kollisolv^{®} MCT 70, MIGLYOL^{®} 810 N, MIGLYOL^{®} 812 N Excipient and Labrafac^{®}, preferably Labrafac^{®}.

In particular, the oily core comprises between 25 and 90% by weight of de Labrafac^{®}, preferably between 60 and 80%, relative to the total weight of the nanocapsule; for example 40, 50, 55, 60 or 70%.

As mentioned above, the shell surrounding the oily core comprises between 3 and 25% by weight based on the total weight of the nanocapsule of at least one lipidic surfactant. In particular, the shell comprises between 3 and 20% by weight relative to the total weight of the nanocapsule of at least one lipidic surfactant, more particularly between 5 and 20%, for example 5, 6, 8, 10, 12 or 20%.

This proportion of lipidic surfactant makes it possible in particular to improve the stability of the nanocapsule during its storage at low temperature. By low temperature is meant here a temperature below 0 ° C.

By "lipidic surfactant" is meant the common sense known to those skilled in the art, i.e. an emulsifying agent having an increased tropism for lipid solutions and making it possible to stabilize the nanocapsule.

In particular, said at least one lipidic surfactant is chosen from lecithins obtained from soybean oil having a percentage of phosphatidylcholine greater than 90% corresponding to the prerequisite of the European Pharmacopoeia, in particular, Lipoïd^{®}, preferably Lipoïd^{®} S100.

More particularly, the shell comprises between 3 and 20% by weight relative to the total weight of the nanocapsule of Lipoïd^{®} S100, more particularly between 5 and 20%, for example 5, 6, 8, 10, 12 or 20%.

As mentioned above, the shell surrounding the oily core also includes at least one hydrophilic surfactant.

By "hydrophilic surfactant" is meant the common sense known to those skilled in the art, i.e. an amphiphilic molecule and/or emulsifying agent allowing and stabilizing the nanoparticles and making the latter soluble in water.

In particular, said at least one hydrophilic surfactant is chosen from nonionic solubilizing and/or emulsifying agents meeting the prerequisite described in the European Pharmacopoeia, in particular Macrogol 15 Hydroxystearate, more particularly Kolliphor^{®}, preferably Kolliphor^{®} HS15.

In particular, the shell comprises between 15 and 60% by weight relative to the total weight of the nanocapsule of at least one hydrophilic surfactant, more particularly between 20 and 50%, for example 15; 20; 30; 35; 40 or 50%.

More particularly, the shell comprises between 15 and 60% by weight relative to the total weight of the nanocapsule of Kolliphor^{®} HS15, more particularly between 20 and 50%, for example 15; 20; 30; 35; 40 or 50%.

In particular, in the context of the present invention, said at least one compound of formula (I) can be comprised in the nanocapsule at a concentration of between 5 and 15 mg/mL, more particularly between 8 and 13 mg/mL, for example of 9, 10 or 11 mg/mL.

This concentration makes it possible in particular to prevent the crystallization of the compound of formula (I) over time while limiting its hydrolysis.

As mentioned above, the nanocapsule according to the invention comprises a ratio by weight relative to the total weight of the nanocapsule between the medium chain triglycerides and said at least one compound of formula (I) of at least 4, in particular of at least 5.

By "at least 4", is meant in particular between 4 and 100, more particularly between 5 and 50, for example 4, 5, 6, 10, 15, 20, 25, 30, 35, 40, 45, 50, 55, 60, 65,70, 75, 80, 85, 90, 95 or 100.

By "at least 5", is meant in particular between 5 and 100, more particularly between 10 and 50, for example 5, 6, 10, 15, 20, 25, 30, 35, 40, 45, 50, 55, 60, 65,70, 75, 80, 85, 90, 95 or 100.

This ratio makes it possible in particular to avoid crystallization of the compound of formula (I).

Thus, a nanocapsule according to the invention can for example be characterized in that it comprises:
- an oily core comprising between 25 and 90% by weight of Labrafac ^{®}, preferably between 60 and 80%, relative to the total weight of the nanocapsule; and
- a shell surrounding the oily core comprising between 3 and 25% by weight relative to the total weight of the nanocapsule of Lipoïd ^{®} S100, and between 15 and 60% by weight relative to the total weight of the nanocapsule of Kolliphor^{®} HS15, more particularly between 20 and 50%, for example 15; 20; 30; 35; 40 or 50%;
   - in which the oily core comprises at least one compound of formula (I) as mentioned above;
   - in which the ratio by weight relative to the total weight of the nanocapsule between the Labrafac ^{®} and said at least one compound of formula (I) is of at least 4, preferably at least 5.

In particular said at least one compound of formula (I) is S-methyl 4-[2-ethoxyethyl(methyl)amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-allyloxyethyl(methyl)amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-benzyloxyethyl(methyl)amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-(m-tolylmethoxy)ethyl]amino]pent-2-ynethioate; or its pharmaceutically acceptable salts or optical isomers, racemates, diastereoisomers, enantiomers or tautomers.

In particular said at least one compound of formula (I) is S-methyl 4-[2-[(3,4-dimethylphenyl)methoxy]ethyl-methyl-amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-[(4-methoxyphenyl)methoxy]ethyl-methyl-amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-[(3,4-dimethoxyphenyl)methoxy]ethyl-methyl-amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-[(3-chlorophenyl)methoxy]ethyl-methyl-amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-[2-[(3-fluorophenyl)methoxy]ethyl-methyl-amino]-4-methyl-pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-(2-pyridylmethoxy)ethyl]amino]pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-(3-pyridylmethoxy)ethyl]amino]pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-(4-pyridylmethoxy)ethyl]amino]pent-2-ynethioate; or its pharmaceutically acceptable salts or optical isomers, racemates, diastereoisomers, enantiomers or tautomers.

In particular said at least one compound of formula (I) is S-methyl 4-((4-(benzyloxy)butyl)(methyl)amino)-4-methylpent-2-ynethioate; or its pharmaceutically acceptable salts or optical isomers, racemates, diastereoisomers, enantiomers or tautomers.

In particular said at least one compound of formula (I) is S-methyl 4-((2-hydroxyethyl)(methyl)amino)-4-methylpent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-(2-naphthylmethoxy)ethyl]amino]pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl-[2-[(2,6,6-trimethylcyclohexen-1- yl)methoxy]ethyl]amino]pent-2-ynethioate; or its pharmaceutically acceptable salts or optical isomers, racemates, diastereoisomers, enantiomers or tautomers.

In particular said at least one compound of formula (I) is S-methyl 2,5,10,11,11-pentamethyl-6-oxo-7-oxa-2,5,10-triazatetradec-12-yne-14-thioate; or its pharmaceutically acceptable salts or optical isomers, racemates, diastereoisomers, enantiomers or tautomers.

In particular said at least one compound of formula (I) is S-methyl 4-methyl-4-[methyl(2-phenoxycyclopentyl)amino]pent-2-ynethioate; or its pharmaceutically acceptable salts or optical isomers, racemates, diastereoisomers, enantiomers or tautomers.

In particular said at least one compound of formula (I) is (S)-S-methyl 4-(2-((benzyloxy)methyl)pyrrolidin-1-yl)-4-methylpent-2-ynethioate; or its pharmaceutically acceptable salts or optical isomers, racemates, diastereoisomers, enantiomers or tautomers.

In particular said at least one compound of formula (I) is S-methyl 4-[3(benzyloxy)-1pyrrolidinyl])-4-methylpent-2-ynethioate; or its pharmaceutically acceptable salts or optical isomers, racemates, diastereoisomers, enantiomers or tautomers.

In particular said at least one compound of formula (I) is S-methyl 1- 4-Dimethylamino-4-methyl-pent-2-ynethioate; or its pharmaceutically acceptable salts or optical isomers, racemates, diastereoisomers, enantiomers or tautomers.

In particular said at least one compound of formula (I) is S-methyl 5- 4-Methyl-4-morpholin-4-yl-pent-2-ynethioate; or its pharmaceutically acceptable salts or optical isomers, racemates, diastereoisomers, enantiomers or tautomers.

In particular said at least one compound of formula (I) is S-methyl 4-methyl-4-[methyl (octyl) amino] pent-2-ynethioate; or its pharmaceutically acceptable salts or optical isomers, racemates, diastereoisomers, enantiomers or tautomers.

### Preparation of the lipidic nanocapsule

The lipidic nanocapsules can be prepared as mentioned in the experimental part. For example, the nanocapsules are produced as follows:
- Weigh the medium chain triglycerides, add the at least one compound of formula (I) and leave under steering for mixing;
- Weigh said at least one hydrophilic surfactant and said at least one lipidic surfactant, add NaCl and water;
- Transfer the aqueous phase into the oily phase under steering;
- Transfer into the oil bath and heat up the solution under strong steering;
- Once a certain temperature is reached, transfer into the ice to cool down ;
- Transfer back to the oil bath to heat;
- Repeat cooling and heating steps additional times;
- At the end of the last heating step, transfer into the ice bath and cool down ;
- When the formulation reaches a certain temperature, add cold water ;
- Remove the ice bath and leave under agitation ;
- Transfer the formulation into an appropriate container.

In one embodiment, the nanocapsule can be formulated in a formulation comprising water and a salt, such as NaCl.

Thus this formulation comprises the nanoparticle, water, preferably between 50 and 70%, and for example 60%, by weight relative to the total weight of the formulation, and a salt, preferably between 1 and 5%, for example 2 or 3%, by weight, relative to the total weight of the composition.

In one embodiment, said compound of formula (I) or said lipidic nanocapsule is comprised in a pharmaceutical composition.

Such composition may be prepared for use in oral administration, particularly in the form of tablets or capsules, in particular orodispersible (lyoc) tablets; or parenteral administration, particularly in the form of liquid solutions, suspensions or emulsions.
It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

### Use

As already mentioned, the present invention relates to at least one compound of formula (I) as herein described for use for modulating the immune response in a subject.

As explained above, this compound can be comprised in a lipidic nanoparticle, and said compound or said lipidic nanoparticule can be comprised in a pharmaceutical composition.

Within the scope of the present invention, by « immunomodulating » properties or allowing « modulation of the immune response » are understood the meaning usually ascribed to these terms and well known to one skilled in the art, in particular any property giving the possibility of stimulating or slowing down the immune reactions of a subject.

In particular, the present invention relates to said at least one compound of formula (I) for use for stimulating the immune response in a subject.

More particularly, the inventors of the present invention have found that compounds of formula (I) are able to boost white blood cells and responses to infections, in particular via stimulation and promotion of maturation and activation of neutrophils.

As such, the present invention relates more particularly to at least one compound of formula (I) for use for treating and/or preventing infectious pathologies, in particular via stimulation and promotion of maturation and/or activation of neutrophils.

By "infectious pathologies" is meant disorders caused by organisms such as bacteria, viruses, fungi or parasites. For example, bacterial infections including *Salmonella, Shigella, E. coli, Staphylococcus aureus, Streptococcus pneumoniae,* and *Haemophilus influenzae,* bacterial meningitidis, chlamydia fungal infections including aspergillosis, fungal meningitis, histoplasmosis, parasitic infections including malaria, toxoplasmosis, trichomoniasis, giardiasis, tapeworm and roundworm infections, leishmaniasis and viral infections including chickenpox, influenza, herpes human papillomavirus, infectious monocucleosis, mups, roseaola, smallpox, fifth disease, measles, rubella and chikungunya virus infection can be cited.

In particular, a compound of formula (I) can be used for the treatment of patients with low neutrophil count to promote neutrophil recovery. Said use can be especially useful in managing patients with drug-induced neutropenia, said drug including chemotherapeutic agents such as alkylating agents, anthracyclines, camptothecins, mitomycin C, taxanes, vinblastine, hydroxyurea, among others and non-chemotherapeutic drugs that induce late-onset neutropenia which have the potential to be a long-lasting complication such as rituximab, carbimazole, clozapine, dapsone, dipyrone, methimazole, oxacillin, vancomycin, penicillin G, procainamide, propylthiouracil, sulfasalazine, and ticlopidine and commonly used drugs that cause neutropenia at lower rates such as angiotensin converting enzyme (ACE) inhibitors, H2 blockers, non-steroidal anti-inflammatory drugs (NSAIDs), and antiarrhythmic drugs, among others.

In addition, the inventors of the present invention have found that the compounds of formula (I) are able to improve wound healing and/or tissue repair; in particular via promotion of angiogenesis, M2 differentiation and production of M2-associated growth factors such as VEGFA and IGF-1, which help tissue regeneration. In addition, they found that said compounds are able to manage autoimmune pathological conditions and/or metabolic diseases, in particular that cause inflammation, in particular via mobilization and recruitment of monocytes to become tissue-resident M2 macrophage.

As such, the present invention also more particularly relates to at least one compound of formula (I) for use for treating and/or preventing wound healing and/or tissue repair; autoimmune pathological conditions and/or metabolic diseases, in particular that cause inflammation.

By "autoimmune pathological conditions" is meant the normal meaning for the man skilled in the art, i.e a condition arising from an abnormal immune response to a functioning body part. The following conditions can be cited as examples: -rheumatoid arthritis, inflammatory bowel disease, systemic lupus erythematosus, multiple sclerosis and psoriasis.

By "metabolic diseases" is meant the normal meaning for the man skilled in the art, i.e a disease in which abnormal chemical reactions in the body alter the normal metabolic process. The following diseases can be cited as examples: atherosclerosis, type 2 diabetes, chronic kidney disease, non-alcoholic fatty liver disease, and obesity, including visceral obesity affecting adipose tissue, liver, skeletal muscle, pancreas, intestines, and hypothalamus.

In one embodiment, the at least one compound of formula (I) is combined with an immune checkpoint inhibitor.

"Immune checkpoint inhibitor" or "ICI" are compounds well known by the man skilled in the art. These compounds are a type of drug that blocks proteins called checkpoints that are made by some types of immune system cells, such as T cells, and some cancer cells.

Preferred immune checkpoint inhibitor according to the invention are chosen from PD-1, PD-L1, PD-L2, CTLA-4, TIGIT, TIM-3, LAG-3 CD160 and 2B4 compounds, in particular from PD1/PDL1 compounds.

The term "patient" or "subject" refers to a warm-blooded animal such as a mammal, in particular a human, male or female, unless otherwise specified, which is afflicted with, or has the potential to be afflicted with one or more diseases and conditions described herein.

The terms "treat", "treating", "treated" or "treatment", as used in the context of the invention, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.

The terms "prevent", "prevention", "preventing" or "prevented", as used in the context of the present invention, refer to the prevention of the onset, recurrence or spread of a disease or disorder, or of one or more symptoms thereof. In certain embodiments, the terms refer to the treatment with or administration of a compound provided herein prior to the onset of symptoms, particularly to patients at risk of disease or disorders provided herein. The terms encompass the inhibition or reduction of a symptom of the particular disease. Subjects with familial history of a disease in particular are candidates for preventive regimens in certain embodiments. In addition, subjects who have a history of recurring symptoms are also potential candidates for the prevention. In this regard, the term "prevention" may be interchangeably used with the term "prophylactic treatment".

In the context of the present invention, the identification of the subjects who are in need of treatment of herein-described diseases and conditions is conducted as above mentioned and is well within the ability and knowledge of the man skilled in the art. A clinician skilled in the art can readily identify, by the above mentioned technics, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

As used herein, an «effective amount" refers to an amount which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The amount of the compound according to the invention, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

Compounds provided herein can be formulated into pharmaceutical compositions, optionally by admixture with one or more pharmaceutically acceptable excipients.

Such compositions may be prepared for use in oral administration, particularly in the form of tablets or capsules, in particular orodispersible (lyoc) tablets; or parenteral administration, particularly in the form of liquid solutions, suspensions or emulsions.
It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers,. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

In particular, when used in combination, said at least one compound of formula (I) and ICI are administered separately, sequentially or simultaneously, preferably separately.

For example, if said at least one compound of formula (I), encapsulated or not, and said ICI are administered sequentially, the at least one compound of formula (I) can be administered prior to administration of the ICI.

In the alternative, the ICI is administered first, followed by the administration of the at least one compound of formula (I).

### Pharmaceutical composition and products

The present invention also relates to a pharmaceutical composition comprising at least one compound of formula (I) as defined above, and an immune checkpoint inhibitor.

The present invention further relates to products comprising at least one compound of formula (I) as defined above and an immune checkpoint inhibitor as a combined preparation for simultaneous use, separate or spread over time as a medicament.

The present invention also relates to at least one compound of formula (I) as defined above, for use as a medicament, wherein said at least one compound of formula (I) is combined with an immune checkpoint inhibitor.

In particular, as described above, said at least one compound of formula (I) can be comprised in a lipidic nanocapsule.

As mentioned above, "immune checkpoint inhibitor" or "ICI" are compounds well known by the man skilled in the art. These compounds are a type of drug that blocks proteins called checkpoints that are made by some types of immune system cells, such as T cells, and some cancer cells.

Preferred immune checkpoint inhibitor according to the invention are chosen from PD-1, PD-L1, PD-L2, CTLA-4, TIGIT, TIM-3, LAG-3 CD160 and 2B4 compounds, in particular from PD1/PDL1 compounds.

More particularly, said pharmaceutical composition, products or at least one compound of formula (I) combined with an ICI are for use in the prevention and/or treatment of cancer.

As used herein and unless otherwise defined, "cancer" refers to the growth, division or proliferation of abnormal cells in the body. It refers to any type of malignant (i.e. non benign) tumor. The malignant tumor may correspond to a primary tumor or to a secondary tumor (i.e. a metastasis). Further, the tumor may correspond to a solid malignant tumor, which includes e.g. carcinomas, adenocarcinomas, sarcomas, melanomas, mesotheliomas, blastomas, or to a blood cancer such as leukemias, lymphomas and myelomas. The cancer may for example correspond to melanoma, breast cancer, lung cancer, prostate cancer, pancreatic cancer, colon cancer, acute myeloid leukemia, hepatocellular carcinoma, squamous cell head & neck cancer, renal cell carcinoma, cervical carcinoma, merkel cell carcinoma, PMBCL, classical Hodgkin lymphoma, gastric and GEJ carcinoma.

In particular, the cancers to prevent and/or to treat are melanoma, lung cancer and colon.

Still particularly, said cancer is a chemoresistant and/or radioresistant cancer.

By "chemoresistant" is meant, a cancer as described herein against which chemotherapy doesn't work or stop working.

By "radioresistant" is meant, a cancer as described herein against which radiotherapy doesn't work or stop working.

In particular, the subject in need of a treatment against cancer is a subject afflicted with such disease.

The term "patient" or "subject" refers to a warm-blooded animal such as a mammal, in particular a human, male or female, unless otherwise specified, which is afflicted with, or has the potential to be afflicted with one or more diseases and conditions described herein.

The terms "treat", "treating", "treated" or "treatment", as used in the context of the invention, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.

The terms "prevent", "prevention", "preventing" or "prevented", as used in the context of the present invention, refer to the prevention of the onset, recurrence or spread of a disease or disorder, or of one or more symptoms thereof. In certain embodiments, the terms refer to the treatment with or administration of a compound provided herein prior to the onset of symptoms, particularly to patients at risk of disease or disorders provided herein. The terms encompass the inhibition or reduction of a symptom of the particular disease. Subjects with familial history of a disease in particular are candidates for preventive regimens in certain embodiments. In addition, subjects who have a history of recurring symptoms are also potential candidates for the prevention. In this regard, the term "prevention" may be interchangeably used with the term "prophylactic treatment".

In the context of the present invention, the identification of the subjects who are in need of treatment of herein-described diseases and conditions is conducted as above mentioned and is well within the ability and knowledge of the man skilled in the art. A clinician skilled in the art can readily identify, by the above-mentioned technics, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

As used herein, an «effective amount" refers to an amount which is effective in reducing, eliminating, treating, or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The amount of the compound according to the invention, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

Compounds provided herein can be formulated into pharmaceutical compositions, optionally by admixture with one or more pharmaceutically acceptable excipients.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

In particular, the at least one compound of formula (I) and ICI are administered separately, sequentially or simultaneously, preferably separately.

For example, if said at least one compound of formula (I), encapsulated or not, and said ICI are administered sequentially, the at least one compound of formula (I) can be administered prior to administration of the ICI.

In the alternative, the ICI is administered first, followed by the administration of the at least one compound of formula (I).

In the context of the present invention, it should be understood that "a compound for use for the treatment or prevention of" is equivalent to "the use of a compound for the treatment or prevention of" and to "the use of a compound for the manufacture of a medicament for the treatment or prevention of ".

The present invention will be further illustrated by the following figures and examples.
*Note: in the following figures and examples, DIMATE corresponds to S-methyl 1-4-Dimethylamino-4-methyl-pent-2-ynethioate.*

### Figures

**Figure 1****:** Illustration of a nanocapsule according to the invention

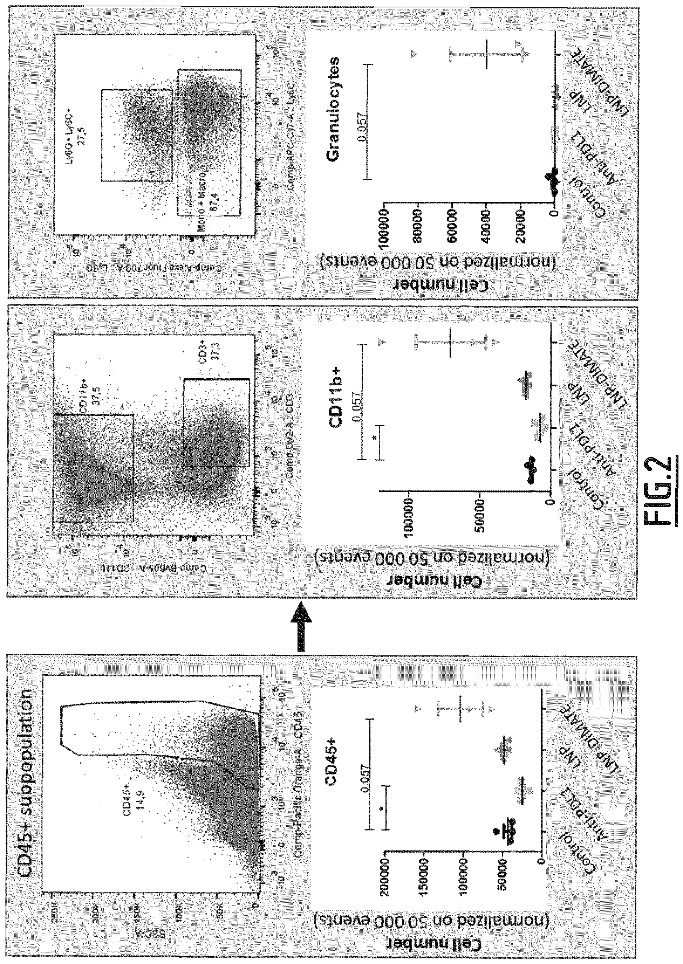

**Figure 2****:** Flow cytometry gating strategy and quantification of neutrophils and granulocyte-macrophage blood cell subpopulations in immunocompetent mice treated with a lipidic nanoparticle formulation loaded with DIMATE (LNP-DIMATE) or unloaded (LNP). Controls consisted in mice treated with PBS (drug vehicle) or with the immune-checkpoint inhibitor anti-PDL1.

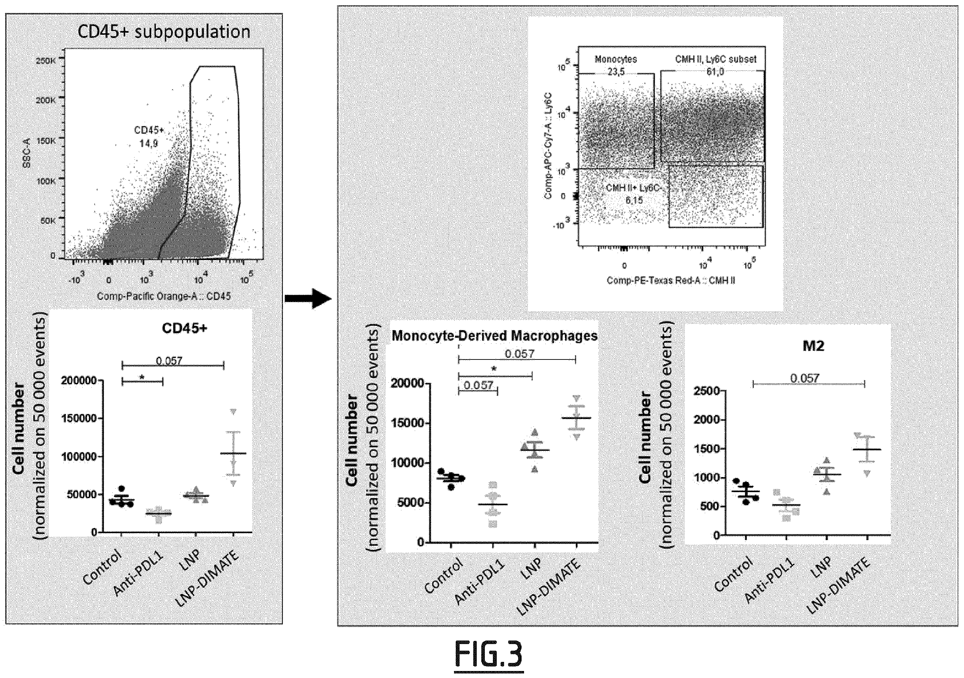

**Figure 3****:** Quantification of monocyte-derived macrophages and macrophages type M2 (M2) blood cell subpopulations in immunocompetent mice treated with a lipidic nanoparticle formulation loaded with DIMATE (LNP-DIMATE) or unloaded (LNP). As in figure 2, controls consisted in mice treated with PBS (drug vehicle) or with the immune-checkpoint inhibitor anti-PDL1.

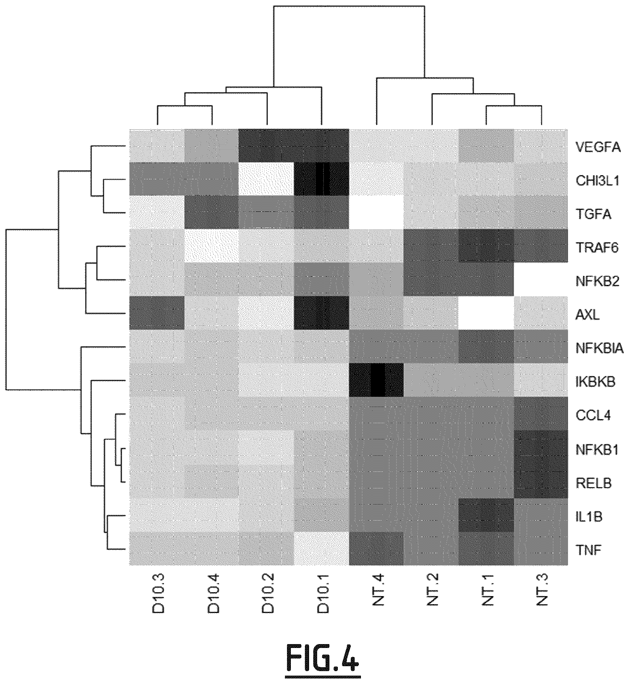

**Figure 4****:** Gene expression profile of genes associated to NFkB signaling pathway (NFKB2, IKBKB, NFKB1, RELB, NFKBIA, TRAF6), M2 (AXL, CHI3L1) as well as with differentiation, cell proliferation and angiogenesis (VEGFA, TGFA) in HL60 cells after treatment with DIMATE compared to non-treated cells. The HL60 cell line is a promyeloblast cell line of lymphoblast-like morphology known to exhibit phagocytic activity and responsiveness to chemotactic stimuli. *On the abscissa axis, samples are separated in two groups (NT: non-treated, D10: DIMATE (10µM)), N=4 independent experiments). On the ordinates, the probes that represent differentially expressed genes).*

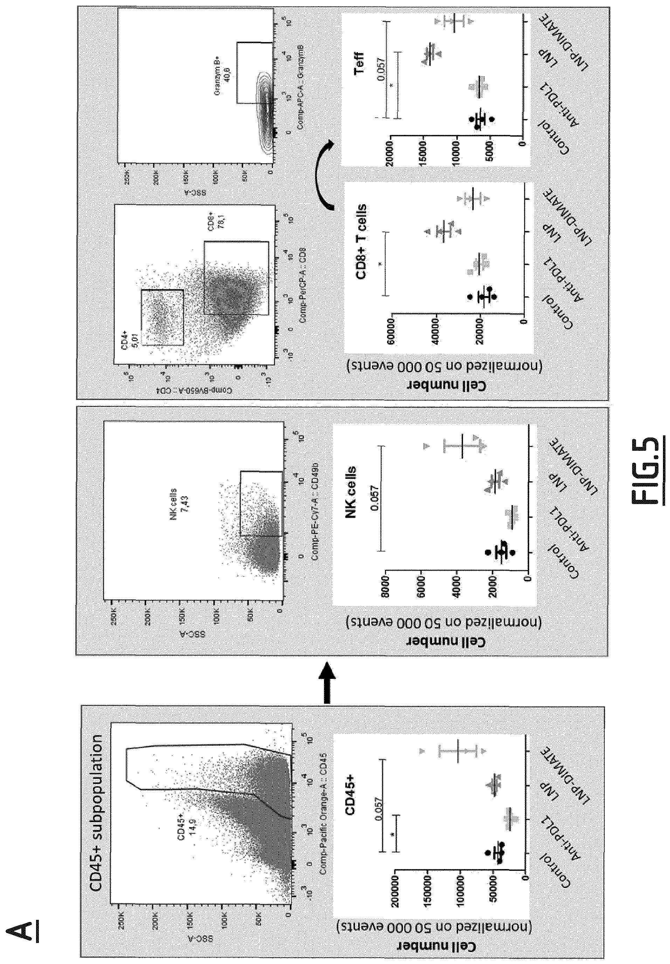

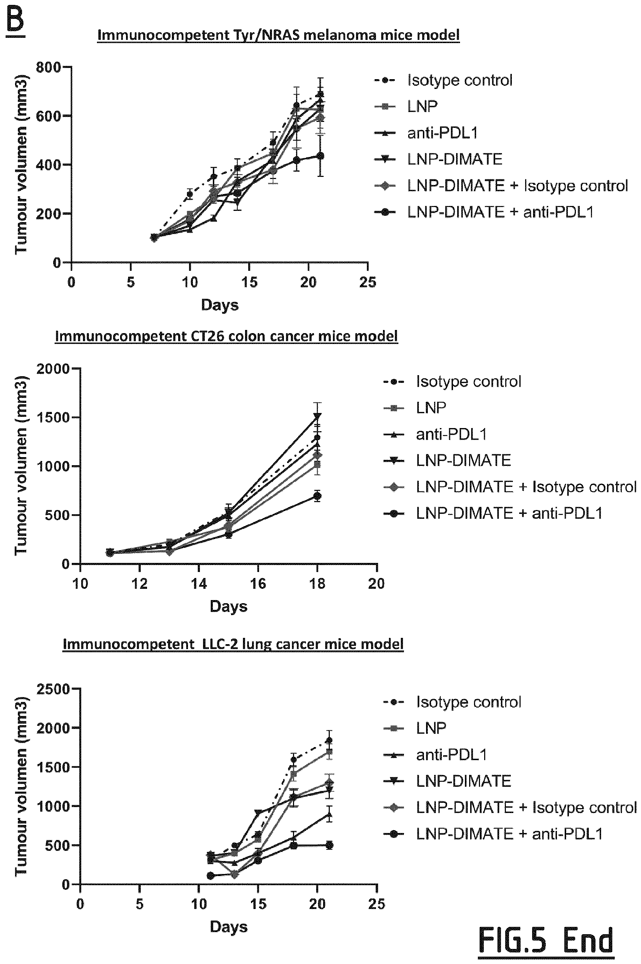

**Figure 5****: A** Quantification of NK cells, CD8⁺ T cells and T effector cell subpopulations in the intra-tumoral environment of a Tyr-BRAF immunocompetent melanoma mice model treated with a lipidic nanoparticle formulation loaded with DIMATE (LNP-DIMATE) or unloaded (LNP). Controls consisted in mice treated with PBS (drug vehicle) or with the immune-checkpoint inhibitor anti-PDL1. **B** Synergistic inhibitory effect of LNP-DIMATE in combination with anti-PDL1 on melanoma, colon cancer and lung cancer tumor growth, in animal models of cancer immunotherapy.

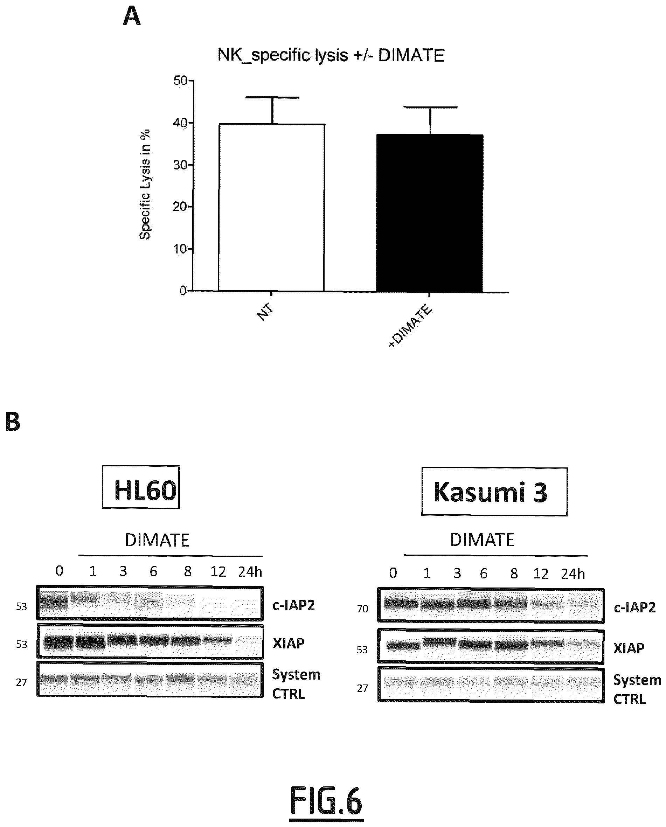

**Figure 6****:** A Lytic activity of human derived-NK cells exposed to DIMATE and expressed as a percentage of dead target cells (k562 cells). N = 6; Scale bars =standard error. **B** c-IAP2 and XIAP protein expression in response to DIMATE in HL60 and kasumi 3 hematopoietic cell lines. Loading control corresponds to the internal system control provided by the WES Protein Simple's manufacturer.

### Examples

### Part 1- Preparation of the compounds of formula (I)

Representative compounds formula (I) are summarized in the table 1 below:

**Table 1**

| Example | Structure | Name |
|---|---|---|
| 1 | 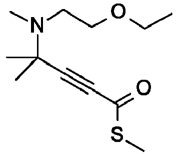 | S-methyl 4-[2-ethoxyethyl (methyl)amino]-4-methyl-pent-2-yneth ioate |
| 2 | 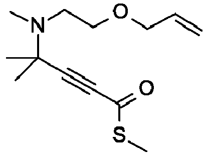 | S-methyl 4-[2-allyloxyethyl(methyl)amino]-4-methyl -pent-2-yneth ioate |
| 3 | 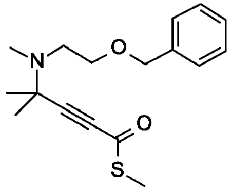 | S-methyl 4-[2-benzyloxyethyl(methyl)amino]-4-methyl-pent-2-yneth ioate |
| 4 | 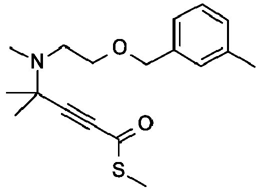 | S-methyl 4-methyl-4-[methyl-[2-(m-tolylmethoxy)ethyl]amino]pent -2-ynethioate |
| 5 | 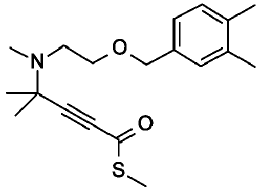 | S-methyl 4-[2-[(3,4-dimethylphenyl) methoxy]ethylmethyl-amino]-4-methyl-pent-2-ynethioate |
| 6 | 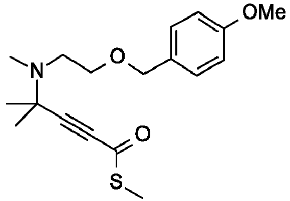 | S-methyl 4-[2-[(4-methoxyphenyl)methoxy]ethylmethyl-amino]-4-methyl-pent-2-ynethioate |
| 7 | 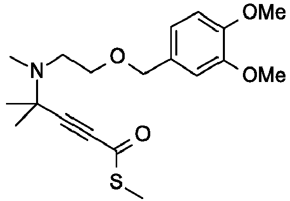 | S-methyl 4-[2-[(3,4-dimethoxyphenyl)methoxy]ethylmethyl-amino]-4-methyl-pent-2-ynethioate |
| 8 | 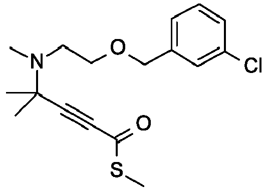 | S-methyl 4-[2-[(3-chlorophenyl)methoxy]ethylmethyl-amino]-4-methyl-pent-2-ynethioate |
| 9 | 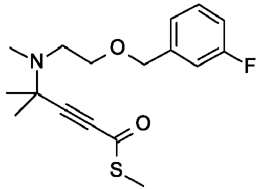 | S-methyl 4-[2-[(3-fluorophenyl)methoxy]ethyl-methyl-amino]-4-methyl-pent-2-ynethioate |
| 10 | 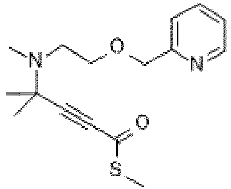 | S-methyl 4-methyl-4-[methyl-[2-(2-pyridylmethoxy)ethyl]amino]pent-2-ynethioate |
| 11 | 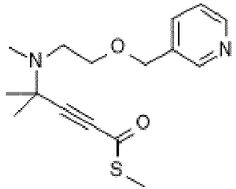 | S-methyl 4-methyl-4-[methyl-[2-(3-pyridylmethoxy)ethyl]amino]pent-2-ynethioate |
| 12 | 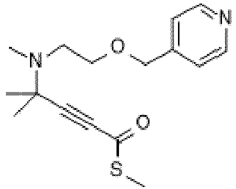 | S-methyl 4-methyl-4-[methyl-[2-(4-pyridylmethoxy)ethyl]amino]pent-2-ynethioate |
| 13 | 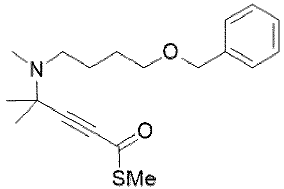 | S-methyl 4-((4-(benzyloxy)butyl)(methyl)amino)-4-methylpent-2-ynethioate |
| 14 | 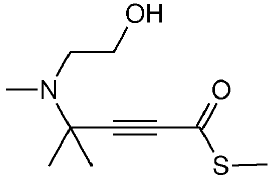 | S-methyl 4-((2-hydroxyethyl)(methyl)amino)-4-methylpent-2-ynethioate |
| 15 | 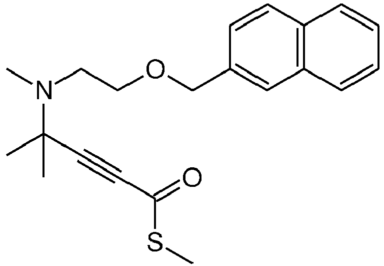 | S-methyl 4-methyl-4-[methyl-[2-(2-naphthylmethoxy)ethyl]amino]pent-2-ynethioate |
| 16 | 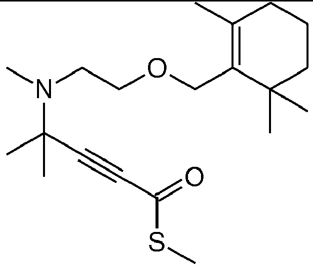 | S-methyl 4-methyl-4-[methyl-[2-[(2,6,6-trimethylcyclohexen-1-yl)methoxy]ethyl]amino]pent -2-ynethioate |
| 17 | 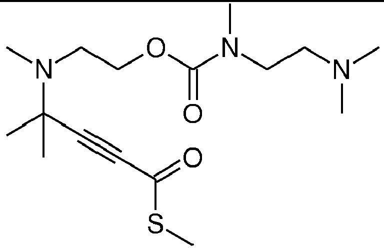 | S-methyl 2,5,10,11,11-pentamethyl-6-oxo-7-oxa-2,5,10-triazatetradec-12-yne-14-thioate |
| 18 | 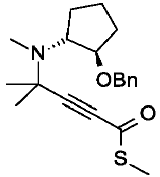 | S-methyl 4-methyl-4-[methyl(2-phenoxycyclopentyl)amino]pent-2-ynethioate |
| 19 | 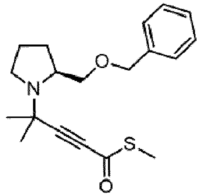 | (S)-S-methyl 4-(2-((benzyloxy)methyl)pyrrolidin-1-yl)-4-methylpent-2-ynethioate |
| 20 | 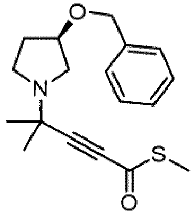 | S-methyl 4-[(3(benzyloxy)-1 pyrrolidinyl])-4-methylpent-2-ynethioate |
| 21 | 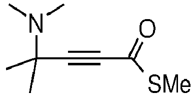 | S-methyl 4-dimethylamino-4-methyl-pent-2-ynethioate |
| 22 | 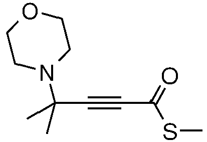 | S-methyl 4-methyl-4-morpholin-4-yl-pent-2-ynethioate |
| 23 | 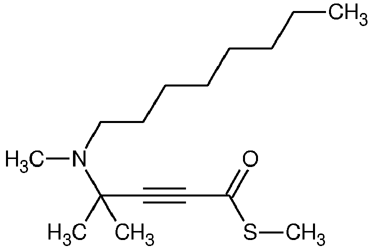 | S methyl 4-methyl-4- [methyl (octyl) amino] pent-2-ynethioate |

Representative compounds of formula (I) can be synthesized according to the following procedures.

### General analytical procedures

The ¹H and ¹³C NMR spectra were recorded on a Bruker Advance ALS300 and DRX400 MHz from Bruker. Chemical shifts are reported in ppm (δ) and were referenced to DMSO-*d6* (¹H, 2.50 ppm; ¹³C, 39.52 ppm) or CDCl3 (7.26 ppm). The coupling constants (*J*) were given in Hz.

The HRMS-ESI mass spectra were recorded in positive-ion mode on a hybrid quadrupole time-of-flight mass spectrometer (MicroTOFQ-II, Bruker Daltonics, Bremen) with an Electrospray Ionization (ESI) ion source. For the mass spectrometry of low resolution, LRMS-ESI mass spectra were recorded in a Thermo Finnigan MAT 95 XL spectrometer.

### Example 1: S-methyl 4-[2-ethoxyethyl(methyl)amino]-4-methyl-pent-2-ynethioate

**Preparation of N-(2-ethoxyethyl))-N,2-dimethyl-but-3-yn-2-amine** : To a solution of N-methyl-N-(2'hydroxyethyl)-3-amino-3methyl-1-butyne (Easton, Nelson R.; Hennion, George F. U.S. (1967), US 3337625 19670822.) (1.0 g, 7.08 mmol) and iodoethane (0.98 mL, 7.6 mmol) in THF (12 mL) was added NaH (0.459 g, 11.5 mmol) at room temperature and the mixture was refluxed for 3 h. Mixture was then carefully hydrolyzed at room temperature by water and extracted by EtOAc (3x25 mL). Combined organic layers were washed with brine, dried over Na₂SO₄ and concentrated in vacuo. Purification of the crude by chromatography on silicagel (petroleum ether/EtOAc=70/30) gave pure N-(2-ethoxyethyl)-N,2-dimethyl-but-3-yn-2-amine (0.479 g, 40%) .
¹H NMR (300 MHz, DMSO) δ 3.45 - 3.36 (m, 4H), 3.11 (s, 1H), 2.51 (t, J = 6.7 Hz,. 2H), 2.20 (s, 3H), 1.27 (s, 6H), 1.09 (t, J = 7.0 Hz, 3H).
ESI-LRMS 170.0 [M+H]+.

**Preparation of S-methyl 4-[2-ethoxyethyl(methyl)amino]-4-methyl-pent-2-ynethioate** : To N-(2-ethoxyethyl)-N,2-dimethyl-but-3-yn-2-amine (0.367 g, 2.17 mmol) in THF (11 mL) was added dropwise a 2.28 M n-BuLi solution in hexane (1.14 mL, 2.60 mmol) at -70°C. After 5 min at -70°C the reaction mixture was warmed to 0°C, maintained 10 min at this temperature then cooled at -70°C before a 30 min bubbling with carbonyl sulfide (COS) through the solution. The yellow solution was warmed to 0°C, stirred for additional 10 min at this temperature before dropwise addition of iodomethane (0.162 mL, 2.60 mmol). The mixture was stirred for 2 h, carefully hydrolyzed at 0°C by water and extracted with ether. Combined organic layers were washed with brine, dried over Na₂SO₄ and concentrated in vacuo. Purification of the crude by chromatography on silicagel (petroleum ether/EtOAc=90/10) gave pure S-methyl 4-[2-ethoxyethyl(methyl)amino]-4-methyl-pent-2-ynethioate (0.369 g, 70%) as an near colorless oil.
¹H NMR (300 MHz, DMSO) δ 3.42 (t, *J* = 6.3 Hz, 2H), 3.42 (q, *J* = 7.0 Hz, 2H), 2.56 (t, *J* = 6.3 Hz, 2H), 2.39 (s, 3H), 2.25 (s, 3H), 1.36 (s, 6H), 1.10 (t, *J =* 7.0 Hz, 3H).
ESI- HRMS calc for C₁₂H₂₂NO₂S [M+H]+: 244.1366, found: 244.1362.

### Example 2: S-methyl 4-[2-alivioxyethyl(methyl)aminol-4-methyl-pent-2-ynethioate

**Preparation of N-(2-allyloxvethyl)-N,2-dimethyl-but-3-yn-2-amine** : To N-methyl-N-(2'hydroxyethyl)-3-amino-3methyl-1-butyne (Easton, Nelson R.; Hennion, George F. U.S. (1967), US 3337625 19670822.)) (1.0 g, 7.08 mmol) in THF (12 mL) was added NaH (0.340 g, 8.50 mmol) at 0°C. After 15 min at 0°C and 15 min at room temperature, *n*-Bu₄NI (0.026 g, 0.071 mmol) was added in one portion at 0°C followed by dropwise addition of allyl bromide (0.735 mL, 8.50 mmol). Reaction mixture was allowed to reach room temperature, stirred overnight, then carefully hydrolyzed by water and extracted by ether (3x25 mL). Combined organic layers were washed with brine (25 mL), dried over Na₂SO₄ and concentrated in vacuo. Purification by chromatography on silicagel (petroleum ether/ether=80/20 to 70/30) gave pure N-(2-allyloxyethyl)-N,2-dimethyl-but-3-yn-2-amine (0.941 g, 73%) as an oil.
¹H NMR (300 MHz, DMSO) δ 5.88 (ddt, J = 17.3, 10.5, 5.3 Hz, 1H), 5.24 (ddd, J = 17.3, 3.8, 1.7 Hz, 1H), 5.16 - 5.09 (m, 1H), 3.93 (dt, J = 5.3, 1.6 Hz, 2H), 3.43 (t, J = 6.4 Hz, 2H), 3.12 (s, 1H), 2.55 (t, J = 6.4 Hz, 2H), 2.21 (s, 3H), 1.27 (s, 6H).
ESI-LRMS 182.0 [M+H]+.

**Preparation of S-methyl 4-[2-allyloxyethyl(methyl)amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-(2-allyloxyethyl)-N,2-dimethyl-but-3-yn-2-amine. Scale: 2.2 mmol. Purification by chromatography on silicagel (petroleum ether/EtOAc=90/10 to 80/20). Yield : 65%. Near colorless oil.
¹H NMR (300 MHz, DMSO) δ 5.88 (ddt, ***J =*** 17.3, 10.5, 5.3 Hz, 1H), 5.24 (ddd, ***J =*** 17.3, 3.8, 1.7 Hz, 1H), 5.17 - 5.10 (m, 1H), 3.94 (dt, *J* = 5.3, 1.5 Hz, 2H), 3.45 (t, *J =* 6.2 Hz, 2H), 2.58 (t, *J* = 6.2 Hz, 2H), 2.39 (s, 3H), 2.26 (s, 3H), 1.36 (s, 6H).
ESI- HRMS calc for C₁₃H₂₂NO₂S [M+H]+: 256.1366, found: 256.1364.

### Example 3: S-methyl 4-[2-benzyloxyethyl)(methyl)amino]-4-methyl-pent-2-ynethioate

**Preparation of N-(2-benzyloxyethyl)-N,2-dimethyl-but-3-yn-2-amine** : The compound is obtained by using the same process as the one described for N-(2-allyloxyethyl)-N,2-dimethyl-but-3-yn-2-amine [example 2] using 1.015 eq of NaH and 1.01 eq. of benzyl bromide. Purification by chromatography on silicagel (petroleum ether/EtOAc=90/10). Yield : 81%. Colorless oil.
¹H NMR (300 MHz, DMSO) δ 7.39 - 7.24 (m, 5H), 4.47 (s, 2H), 3.49 (t, J = 6.3 Hz, 2H), 3.12 (s, 1H), 2.58 (t, J = 6.3 Hz, 2H), 2.21 (s, 3H), 1.27 (s, 6H). ESI-LRMS 232.0 [M+H]+.

**Preparation of S-methyl 4-[2-benzyloxyethyl(methyl)amino]-4-methyl-pent-2-ynethioate** : The compound is obtained by using the same process as the one described for S-methyl 4-[2-ethoxyethyl(methyl)amino]-4-methyl-pent-2-ynethioate [example 1] starting from N-(2-benzyloxyethyl)-N,2-dimethyl-but-3-yn-2-amine. Scale: 2.2 mmol. Purification by chromatography on silicagel (petroleum ether/EtOAc=90/10). Yield : 79%. Colorless oil.
¹H NMR (300 MHz, DMSO) δ 7.37 - 7.26 (m, 5H), 4.48 (s, 2H), 3.52 (t, *J* = 6.1 Hz, 2H), 2.62 (t, *J* = 6.1 Hz, 2H), 2.38 (s, 3H), 2.26 (s, 3H), 1.36 (s, 6H).
ESI- HRMS calc for C₁₇H₂₄NO₂S [M+H]+: 306.1522, found: 306.1514.

*Alternative protocol:* To N-(2-benzyloxyethyl)-N,2-dimethyl-but-3-yn-2-amine (0.650 g, 2.81 mmol) in THF (8 mL) was added dropwise a 2.28 M *n*-BuLi solution in hexane (1.36 mL, 3.09 mmol) at -70°C. After 5 min at -70°C the reaction mixture was warmed to 0°C, maintained 30 min at this temperature and CO₂ was bubbled through the solution for 30 min. The mixture was warmed to room temperature within 5 min then re-cooled at 0°C. Isobutyl chloroformate (0.40 ml, 3.08 mmol) was added dropwise and the mixture stirred for 10 min before addition of sodium methoxide (0.236 g, 3.37 mmol) in one portion. The mixture was warmed to room temperature stirred for additional 15 min at this temperature then carefully hydrolyzed at 0°C by water and extracted with ether. Combined organic layers were washed with brine, dried over Na₂SO₄ and concentrated in vacuo. Purification of the crude by chromatography on silicagel (petroleum ether/EtOAc=90/10) gave pure S-methyl 4-[2-ethoxyethyl(methyl)amino]-4-methyl-pent-2-ynethioate (0.307 g, 36%).

### Example 4: S-methyl 4-methyl-4-[methyl-[2-(m-tolylmethoxy)ethyl]amino]pent-2-ynethioate

**Preparation of N-2-dimethyl-N-[2-(m-tolylmethoxy)ethyl]but-3-yn-2-amine:** The compound is obtained by using the same process as the one described for N-(2-allyloxyethyl)-N,2-dimethyl-but-3-yn-2-amine [example 2] using 3-Methylbenzyl bromide. Purification by chromatography on silicagel (petroleum ether/EtOAc=90/10). Scale : 4.5 mmol. Yield : 79%. Colorless oil.
¹H NMR (300 MHz, DMSO) δ 7.26 - 7.19 (m, 1H), 7.16 - 7.05 (m, 3H), 4.43 (s, 2H), 3.48 (t, J = 6.3 Hz, 2H), 3.12 (s, 1H), 2.57 (t, J = 6.3 Hz, 2H), 2.30 (s, 3H), 2.21 (s, 3H), 1.27 (s, 6H).
ESI-LRMS 246.1 [M+H]+.

**Preparation of S-methyl 4-methyl-4-[methyl-[2-(m-tolylmethoxy)ethyl]amino]pent-2-ynethioate** : The compound is obtained by using the same process as the one described for S-methyl 4-[2-ethoxyethyl(methyl)amino]-4-methyl-pent-2-ynethioate [example 1] starting from N-2-dimethyl-N-[2-(m-tolylmethoxy)ethyl]but-3-yn-2-amine. Scale : 1.3 mmol. Purification by chromatography on silicagel (petroleum ether/EtOAc=90/10). Yield : 77%. Colorless oil.
¹H NMR (300 MHz, DMSO) δ 7.26 - 7.19 (m, 1H), 7.16 - 7.05 (m, 3H), 4.44 (s, 2H), 3.50 (t, *J =* 6.1 Hz, 2H), 2.62 (t, *J =* 6.1 Hz, 2H), 2.38 (s, 3H), 2.30 (s, 3H), 2.26 (s, 3H), 1.36 (s, 6H).
ESI- HRMS calc for C₁₈H₂₆NO₂S [M+H]+: 320.1679, found: 320.1667.

### Example 5: S-methyl 4-[2-[(3,4-dimethylphenyl)methoxy]ethyl-methyl-amino]-4-methyl-pent-2-ynethioate

**Preparation of N-[2-[(3,4-dimethylphenyl)methoxy]ethyl]-N,2-dimethyl-but-3-yn-2-amine** : The compound is obtained by using the same process as the one described for N-(2-allyloxyethyl)-N,2-dimethyl-but-3-yn-2-amine [example 2] using 3,4-Dimethylbenzyl bromide. Purification by chromatography on silicagel (petroleum ether/EtOAc=60/40). Scale : 3.8 mmol. Yield : 60%. Colorless oil.
¹H NMR (300 MHz, DMSO) δ 7.12 - 7.06 (m, 2H), 7.04 - 6.99 (m, 1H), 4.39 (s, 2H), 3.45 (t, J = 6.3 Hz, 2H), 3.12 (s, 1H), 2.56 (t, J = 6.3 Hz, 2H), 2.20 (s, 6H), 2.19 (s, 3H), 1.27 (s, 6H).
ESI-LRMS 182.0 [M+H]+. ESI-LRMS 260.0 [M+H]+.

**Preparation of S-methyl 4-[2-[(3,4-dimethylphenyl)methoxy]ethyl-methyl-amino]-4-methyl-pent-2-ynethioate** : The compound is obtained by using the same process as the one described for S-methyl 4-[2-ethoxyethyl(methyl)amino]-4-methyl-pent-2-ynethioate [example 1] starting from N-[2-[(3,4-dimethylphenyl)methoxy]ethyl]-N,2-dimethyl-but-3-yn-2-amine. Scale: 1.3 mmol. Purification by chromatography on silicagel (petroleum ether/EtOAc=90/10). Yield : 77%. Near colorless oil.
¹H NMR (300 MHz, DMSO) δ 7.12 - 7.06 (m, 2H), 7.05 - 6.99 (m, 1H), 4.40 (s, 2H), 3.48 (t, *J* = 6.2 Hz, 2H), 2.60 (t, *J* = 6.2 Hz, 2H), 2.38 (s, 3H), 2.25 (s, 3H), 2.20 (s, 3H), 2.19 (s, 3H), 1.36 (s, 6H).
ESI- HRMS calc for C₁₉H₂₈NO₂S [M+H]+: 334.1835, found: 334.1825.

### Example 6: S-methyl 4-[2-[(4-methoxyphenyl)methoxy]ethyl-methyl-amino]-4-methyl-pent-2-ynethioate

**Preparation of N-[2-[(4-methoxyphenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 1-(Bromomethyl)-4-methoxybenzene. Purification by chromatography on silicagel (DCM/MeOH=99/1 to 97.5/2.5). Scale : 4.0 mmol. Yield : 53%. Colorless oil.
1H NMR (300 MHz, DMSO) δ 7.27 - 7.20 (m, 2H), 6.93 - 6.86 (m, 2H), 4.39 (s, 2H), 3.74 (s, 3H), 3.45 (t, J = 6.3 Hz, 2H), 3.12 (s, 1H), 2.55 (t, J = 6.4 Hz, 2H), 2.20 (s, 3H), 1.27 (s, 6H).
ESI-LRMS 261.9 [M+H]+.

**Preparation of S-methyl 4-[2-[(4-methoxyphenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(4-methoxyphenyl)methoxy]ethyl]-N,2-dimethyl-but-3-yn-2-amine. Scale: 1.3 mmol. Purification by chromatography on silicagel (petroleum ether/EtOAc=80/20). Yield : 74%. Near colorless oil.
¹H NMR (300 MHz, DMSO) δ 7.27 - 7.21 (m, 2H), 6.93 - 6.87 (m, 2H), 4.40 (s, 2H), 3.74 (s, 3H), 3.48 (t, *J =* 6.2 Hz, 2H), 2.60 (t, *J =* 6.2 Hz, 2H), 2.38 (s, 3H), 2.25 (s, 3H), 1.36 (s, 6H).
ESI- HRMS calc for C₁₈H₂₆NO₃S [M+H]+: 336.1628, found: 336.1613.

### Example 7: S-methyl 4-[2-[(3,4-dimethoxyphenyl)methoxy]ethyl-methyl-amino]-4-methyl-pent-2-ynethioate

**Preparation of N-[2-[(3,4-dimethoxyphenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 4-(Bromomethyl)-1,2-dimethoxybenzene. Purification by chromatography on silicagel (DCM/MeOH=99/1 to 97.5/2.5). Scale : 4.0 mmol. Yield : 67%. Colorless oil.
¹H NMR (300 MHz, DMSO) δ 6.94 - 6.88 (m, 2H), 6.87 - 6.80 (m, 1H), 4.39 (s, 2H), 3.74 (s, 3H), 3.73 (s, 3H), 3.46 (t, J = 6.3 Hz, 2H), 3.12 (s, 1H), 2.56 (t, J = 6.3 Hz, 2H), 2.21 (s, 3H), 1.27 (s, 6H).
ESI-LRMS 292.0 [M+H]+.

**Preparation of S-methyl 4-[2-[(3,4-dimethoxyphenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3,4-dimethoxyphenyl)methoxy]ethyl]-N,2-dimethyl-but-3-yn-2-amine. Scale: 1.0 mmol. Purification by chromatography on silicagel (petroleum ether/EtOAc=90/10). Yield : 67%. Near colorless oil.
¹H NMR (300 MHz, DMSO) δ 6.94 - 6.87 (m, 1H), 6.87 - 6.81 (m, 1H), 4.40 (s, 2H), 3.74 (s, 3H), 3.73 (s, 3H), 3.48 (t, *J =* 6.1 Hz, 2H), 2.61 (t, *J =* 6.2 Hz, 2H), 2.38 (s, 3H), 2.26 (s, 3H), 1.36 (s, 6H).
ESI- HRMS calc for C₁₉H₂₈NO₄S [M+H]+: 366.1734, found: 336.1720.

### Example 8: S-methyl 4-[2-[(3-chlorophenyl)methoxy]ethyl-methyl-amino]-4-methyl-pent-2-ynethioate

**Preparation of N-[2-[(3-chlorophenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 3-Chlorobenzyl bromide. Purification by chromatography on silicagel (petroleum ether/EtOAc=70/30). Scale : 4.0 mmol. Yield : 71%. Colorless oil.
¹H NMR (300 MHz, DMSO) δ 7.43 - 7.25 (m, 4H), 4.49 (s, 2H), 3.50 (t, J = 6.2 Hz, 2H), 3.12 (s, 1H), 2.58 (t, J = 6.2 Hz, 2H), 2.22 (s, 3H), 1.28 (s, 6H).
ESI-LRMS 266.0 [M+H]+.

**Preparation of S-methyl 4-[2-[(3-chlorophenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3-chlorophenyl)methoxy]ethyl]-N,2-dimethyl-but-3-yn-2-amine except that the reaction mixture was maintained at -70°C after n-BuLi addition for 30 min before COS bubbling. Scale : 1.3 mmol. Purification by chromatography on silicagel (petroleum ether/EtOAc=75/25). Yield : 63%. Near colorless oil.
¹H NMR (300 MHz, DMSO) δ 7.44 - 7.24 (m, 4H), 4.50 (s, 2H), 3.52 (t, *J* = 6.0 Hz, 2H), 2.63 (t, *J* = 6.0 Hz, 2H), 2.38 (s, 3H), 2.27 (s, 3H), 1.37 (s, 6H).
ESI- HRMS calc for C₁₇H₂₃ClNO₂S [M+H]+: 340.1133, found: 340.1120.

### Example 9: S-methyl 4-[2-[(3-fluorophenyl)methoxy]ethyl-methyl-amino]-4-methyl-pent-2-ynethioate

**Preparation of N-[2-[(3-fluorophenyl)methoxy]ethyl]-N,2-dimethyl-but-3-yn-2-amine** : The compound is obtained by using the same process as the one described for N-(2-allyloxyethyl)-N,2-dimethyl-but-3-yn-2-amine [example 2] using 3-fluorobenzyl bromide. Purification by chromatography on silicagel (petroleum ether/EtOAc=80/20). Scale : 4.0 mmol. Yield : 71%. Colorless oil.

**Preparation of S-methyl 4-[2-[(3-fluorophenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3-fluorophenyl)methoxy]ethyl]-N,2-dimethyl-but-3-yn-2-amine except that the reaction mixture was maintained at -70°C after *n*-BuLi addition for 30 min before COS bubbling. Scale : 1.3 mmol. Purification by chromatography on silicagel (petroleum ether/EtOAc=70/30). Yield : 87%. Near colorless oil.
¹H NMR (300 MHz, DMSO) δ 7.44 - 7.34 (m, 1H), 7.20 - 7.05 (m, 3H), 4.51 (s, 2H), 3.53 (t, *J =* 6.1 Hz, 2H), 2.63 (t, *J* = 6.1 Hz, 2H), 2.38 (s, 3H), 2.27 (s, 3H), 1.37 (s, 6H).
ESI- HRMS calc for C₁₇H₂₃FNO₂S [M+H]+: 324.1428, found: 324.1415

### Example 10: S-methyl 4-methy)-4-[methy]-[2-(2-pyridylmethoxy)ethyl]amino]pent-2-ynethioate

**Preparation of N-2-dimethyl-N-[2-(2-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 2-(Bromomethyl)pyridine hydrobromide and using 4 eq of NaH. Purification by chromatography on silicagel (DCM/MeOH=99/1 to 95/5). Scale : 2.1 mmol. Yield : 71%. Yellow oil.
¹H NMR (300 MHz, DMSO) δ 8.50 (ddd, J = 4.8, 1.8, 0.9 Hz, 1H), 7.80 (td, J = 7.7, 1.8 Hz, 1H), 7.44 (d, J = 7.8 Hz, 1H), 7.32 - 7.24 (m, 1H), 4.55 (s, 2H), 3.56 (t, J = 6.2 Hz, 2H), 3.13 (s, 1H), 2.61 (t, J = 6.2 Hz, 2H), 2.23 (s, 3H), 1.28 (s, 6H).
ESI-LRMS 233.1 [M+H]+.

**Preparation of S-methyl 4-methyl-4-[methyl-[2-(2-pyridylmethoxy)ethyl]amino]pent-2-ynethioate:** The compound is obtained by using the same processas the one described for S-methyl 4-[2-ethoxyethyl(methyl)amino]-4-methyl-pent-2-ynethioate [example 1] starting from N-2-dimethyl-N-[2-(2-pyridylmethoxy)ethyl]but-3-yn-2-amine and using 1.5 eq of n-BuLi,1.5 eq of Mel and DCM extractions. Scale: 0.9 mmol. Purification by chromatography on silicagel (DCM/MeOH=99/1 to 90/10). Yield : 18%. Yellow oil.
¹H NMR (300 MHz, DMSO) δ 8.54 - 8.48 (m, 1H), 7.80 (td, J = 7.7, 1.8 Hz, 1H), 7.44 (d, J = 7.8 Hz, 1H), 7.28 (dd, J = 6.7, 5.1 Hz, 1H), 4.56 (s, 2H), 3.59 (t, J = 6.1 Hz, 2H), 2.65 (t, J = 6.1 Hz, 2H), 2.38 (s, 3H), 2.28 (s, 3H), 1.37 (s, 6H).).
ESI- HRMS calc for C₁₆H₂₃N₂O₂S [M+H]+: 3071475, found: 307.1471.

### Example 11: S-methyl 4-methyl-4-[methyl-[2-(3-pyridylmethoxy)ethyl]amino]pent-2-ynethioate

**Preparation of N-2-dimethyl-N-[2-(3-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 3-(Bromomethyl)pyridine hydrobromide and using 4 eq of NaH. Purification by chromatography on silicagel (DCM/MeOH=99/1 to 95/5). Scale : 2.1 mmol. Yield : 67%. Yellow oil.
¹H NMR (300 MHz, DMSO) δ 8.56 - 8.52 (m, 1 H), 8.49 (dd, J = 4.8, 1.7 Hz, 1 H), 7.78 - 7.70 (m, 1H), 7.38 (ddd, *J* = 7.8, 4.8, 0.8 Hz, 1H), 4.52 (s, 2H), 3.52 (t, *J =* 6.2 Hz, 2H), 3.12 (s, 1H), 2.58 (t, *J =* 6.2 Hz, 2H), 2.21 (s, 3H), 1.27 (s, 6H).
ESI-LRMS 233.1 [M+H]⁺.

**Preparation of S-methyl 4-methyl-4-[methyl-[2-(3-pyridylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N-2-dimethyl-N-[2-(3-pyridylmethoxy)ethyl]but-3-yn-2-amine and using 1.5 eq of n-BuLi,1.5 eq of Mel and DCM extractions. Scale : 0.4 mmol. Purification by chromatography on silicagel (DCM/MeOH=99/1 to 95/5). Yield : 15%. Yellow oil.
¹H NMR (300 MHz, DMSO) δ 8.54 (d, J = 1.5 Hz, 1H), 8.49 (dd, J = 4.8, 1.7 Hz, 1H), 7.78 - 7.70 (m, 1H), 7.38 (ddd, J = 7.8, 4.8, 0.8 Hz, 1H), 4.53 (s, 2H), 3.54 (t, J = 6.1 Hz, 2H), 2.63 (t, J = 6.1 Hz, 2H), 2.38 (s, 3H), 2.26 (s, 3H), 1.36 (s, 6H) ).
ESI- HRMS calc for C₁₆H₂₃N₂O₂S [M+H]+: 3071475, found: 307.1474.

### Example 12: S-methyl 4-methyl-4-[methyl)-[2-(4-pyridylmethoxy)ethyl]aminol]pent-2-ynethioate

**Preparation of N-2-dimethyl-N-[2-(4-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 4-(Bromomethyl)pyridine hydrobromide and using 4 eq of NaH. Purification by chromatography on silicagel (DCM/MeOH=99/1 to 95/5). Scale : 2.1 mmol. Yield : 95%. Yellow oil.
¹H NMR (300 MHz, DMSO) δ 8.56 - 8.49 (m, 2H), 7.38 - 7.27 (m, 2H), 4.54 (s, 2H), 3.53 (t, J = 6.2 Hz, 2H), 3.13 (s, 1H), 2.61 (t, J = 6.2 Hz, 2H), 2.23 (s, 3H), 1.28 (s, 6H). ESI-LRMS 233.1 [M+H]+.

**Preparation of S-methyl 4-methyl-4-[methyl-[2-(4-pyridylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N-2-dimethyl-N-[2-(4-pyridylmethoxy)ethyl]but-3-yn-2-amine and using 1.5 eq of n-BuLi,1.5 eq of Mel and DCM extractions. Scale : 1.0 mmol. Purification by chromatography on silicagel (DCM/MeOH=99/1 to 95/15). Yield : 24%. Yellow oil.
¹H NMR (300 MHz, DMSO) δ 8.60 - 8.45 (m, 2H), 7.37 - 7.26 (m, 2H), 4.55 (s, 2H), 3.56 (t, J = 6.0 Hz, 2H), 2.65 (t, J = 6.0 Hz, 2H), 2.38 (s, 3H), 2.28 (s, 3H), 1.37 (s, 6H).
ESI- HRMS calc for C₁₆H₂₃N₂O₂S [M+H]+: 3071475, found: 307.1470.

### Example 13: S-methyl 4-((4-(benzyloxy)butyl)(methyl)amino)-4-methylpent-2-vnethioate

**Preparation of 4-(methyl(2-methylbut-3-yn-2-yl)amino)butan-1-ol** : This compound was prepared by standard protocols previously described for the synthesis of N-methyl-N-(2'hydroxyethyl)-3-amino-3methyl-1-butyne (Easton, Nelson R.; Hennion, George F. U.S. (1967), US 3337625 19670822.) starting from commercially available 4-(methylamino)butan-1-ol. 4-(methyl(2-methylbut-3-yn-2-yl)amino)butan-1-ol was obtained as a bright yellow oil. Scale 3 mmol. Yield : 99%.
¹H NMR (300MHz, DMSO) δ 4.41 (t, J = 5.2Hz, 1H), 3.42 - 3.33 (m, 2H), 3.09 (s, 1H), 2.38 - 2.29 (m, 2H), 2.14 (s, 3H), 1.45 - 1.36 (m, 4H), 1.27 (s, 6H).
ESI - LRMS : 170.1 [M+H]+

**Preparation of N-(4-(benzyloxy)butyl)-N,2-dimethylbut-3-yn-2-amine** : The compound was obtained by using the same process as the one described for N-(2-allyloxyethyl)-N,2-dimethyl-but-3-yn-2-amine [example 2] starting from 4-(methyl(2-methylbut-3-yn-2-yl)amino)butan-1-ol and using 1.015 eq of NaH and 1.01 eq. of benzyl bromide. Purification by chromatography on silicagel (cyclohexane/EtOAc=80/20). Scale 2.4 mmol. Yield : 55%. Yellow oil.
¹H NMR (300MHz, DMSO) δ 7.39 - 7.22 (m, 5H), 4.44 (s, 2H), 3.42 (t, J = 6.3Hz, 2H), 3.08 (s, 1H), 2.34 (t, J = 6.9Hz, 2H), 2.13 (s, 3H), 1.60 - 1.37 (m, 4H), 1.26 (s, 6H).
ESI - LRMS : 260.2 [M+H]+

**Preparation of S-methyl 4-((4-(benzyloxy)butyl)(methyl)amino)-4-methylpent-2-ynethioate** : The compound was obtained by using the same process as the one described for S-methyl 4-[2-ethoxyethyl(methyl)amino]-4-methyl-pent-2-ynethioate [example 1] starting from N-(4-benzyloxybutyl)-N,2-dimethyl-but-3-yn-2-amine Purification by chromatography on silicagel (cyclohexane/EtOAc= 90/10). Scale 0.77 mmol. Yield : 80%. Yellow oil.
1H NMR (300MHz, CDCl3) δ 7.38 - 7.19 (m, 5H), 4.48 (s, 2H), 3.47 (t, J = 6.1Hz, 2H), 2.45 (t, J = 6.9Hz, 2H), 2.35 (s, 3H), 2.26 (s, 3H), 1.73 - 1.48 (m, 4H), 1.39 (s, 6H).
ESI - HRMS : calc for C19H28NO2S [M+H]+ 334.1835, found 334.1840.

### Example 14: S-methyl 4-((2-hydroxyethyl)(methyl)amino)-4-methylpent-2-ynethioate

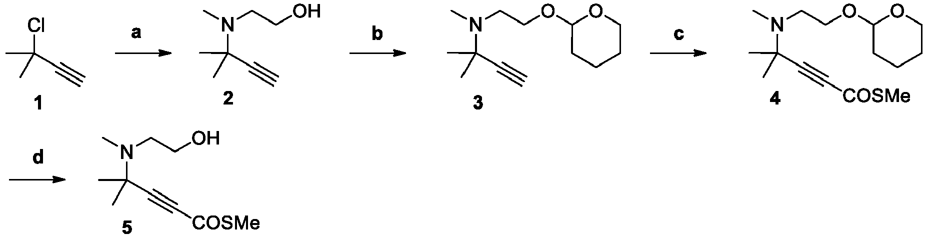

**a)** see Easton, Nelson R.; Hennion, George F. , U.S. (1966), US 3285913 **b**) 3,4-DHP, pTSA, DCM (70%) **c**) nBuLi, THF, -70°C, carbonyl sulfide then Mel 0°C (59%) **d**) pTSA, MeOH, room temperature (90%)

**Preparation of Compound 3: N,2-dimethyl-N-(2-((tetrahydro-2H-pyran-2-yl)oxy)ethyl)but-3-yn-2-amine:** To 2-(methyl(2-methylbut-3-yn-2-yl)amino)ethanol **2** (3.00 g, 21.2 mmol) and 3,4-Dihydro-2H-pyran (5.0 eq) in anhydrous DCM (135 mL) was added p-toluenesulfonic acid (0.1 eq) at room temperature. The reaction mixture was stirred overnight, washed with aqueous saturated NaHCO₃ (30 mL) then brine (30 mL). The organic layer was dried over sodium sulfate, filtered and concentrated *in vacuo.* The residue was first purified by short-path distillation using Kugelrohr apparatus (10-12 Torrs, oven 155°C) then by flash chromatography on silica gel (petroleum ether/ethyl acetate 95/5 to 60/40) to give compound **3** as an oil (yield 70%).
¹H NMR (300 MHz, DMSO) δ 4.60 - 4.50 (m, 1H), 3.82-3.70 (m, 1H), 3.59-3.56 (m, 1H), 3.49 - 3.35 (m, 2H), 3.12 (s, 1H), 2.55 (t, *J =* 6.5 Hz, 2H), 2.21 (s, 3H), 1.77 - 1.35 (m, 6H), 1.27 (s, 6H).

**Preparation of Compound 4: S-methyl 4-methyl-4-(methyl(2-((tetrahydro-2H-pyran-2-yl)oxy) ethyl) amino) pent-2-ynethioate:** To the acetylenic amine **3** (1.00 g, 4.44 mmol) in anhydrous THF (22 mL) was added n-Butyllithium solution (2.2 M in hexanes, 1.5 eq) dropwise. The mixture was allowed to reach to 0°C within 10 minutes then re-cooled to -70°C before carbonyl sulfide bubbling. After 30 minutes the bright yellow solution was carefully warmed to 0°C, stirred 30 minutes at this temperature and methyl iodide (1.2 eq) was added dropwise. The reaction mixture was stirred for 2 hours at 0°C before hydrolysis by water. Extractive work-up by DCM (washing with brine, drying with sodium sulfate and concentration under reduced pressure) gave a crude which was purified by chromatography on silica gel (petroleum ether/ethyl acetate 90/10 to 60/40) to give compound **4** as an oil (yield 59%).
¹H NMR (300 MHz, DMSO) δ 4.58 (t, *J =* 3.2 Hz, 1H), 3.75 (ddd, *J =* 11.4, 7.9, 3.3 Hz, 1H), 3.70 - 3.60 (m, 1H), 3.49 - 3.38 (m, 2H), 2.59 (t, *J* = 6.3 Hz, 2H), 2.39 (s, 3H), 2.27 (s, 3H), 1.77 - 1.39 (m, 6H), 1.36 (s, 6H). ¹³C NMR (75 MHz, CDCl₃) δ 176.62 (C=O), 98.94 (CH), 96.46 (C), 80.97 (C), 66.55 (CH₂), 62.37 (CH₂), 55.19 (C), 52.43 (CH₂), 37.92 (CH₃), 30.75 (CH₂), 28.01 (2xCH₃), 25.59 (CH₂), 19.63 (CH₂), 12.61 (CH₃).

**Preparation of Compound 5: S-methyl 4-((2-hydroxyethyl)(methyl)amino)-4-methylpent-2-ynethioate:** To the aminothiolester **4** (1.00 g, 3.34 mmol) in methanol (15 mL) was added p-toluenesulfonic acid (1.1 eq) at room temperature. The reaction mixture was stirred overnight, washed with aqueous saturated NaHCO₃ (30 mL) then brine (30 mL). The organic layer was dried over sodium sulfate, filtered and concentrated *in vacuo.* The residue was purified by flash chromatography on silica gel (petroleum ether/ethyl acetate 80/20 to 20/80) to give compound **5** as an oil (yield 90%).
¹H NMR (300 MHz, DMSO) δ 4.42 (t, *J =* 5.6 Hz, 1H), 3.44 (td, *J* = 6.7, 5.6 Hz, 2H), 2.46 (, *J* = 6.7 Hz, 2H), 2.39 (s, 3H), 2.24 (s, 3H), 1.36 (s, 6H). ¹³C NMR (75 MHz, CDCl₃) δ 176.46 (C=O), 95.56 (C), 80.89 (C), 58.92 (CH₂), 54.99 (C) , 53.52 (CH₂), 36.12 (CH₃), 27.88 (2xCH₃), 12.53(CH₃). ESI-HRMS: Calc. for C₁₀H₁₈NO₂S [M+H]+ 216.1053 found 216.1043.

### Example 15. S-methyl 4-methy)-4-[methyl-4-(2-naphthylmethoxy)ethyl]amino]pent-2-ynethioate

**Preparation of N,2-dimethyl-N-[2-(2-naphthylmethoxy)ethyl]but-3-yn-2-amine:** The compound is obtained by using the same process as the one described for N-(2-allyloxyethyl)-N,2-dimethyl-but-3-yn-2-amine [example 2] using 1.015 eq of NaH and 1.01 eq. of 2-naphtyl bromide. Purification by chromatography on silicagel (petroleum ether/EtOAc=90/10). Yield: 61%. orange oil.
1H NMR (300 MHz, DMSO) δ 7.91 - 7.88 (m, 4H), 7.50 - 7.48 (m, 3H), 4.65 (s, 2H), 3.55 (t, 6.1Hz, 2H), 3.13 (s, 1H), 2.62 (t, 6.2Hz, 2H), 2.23 (s, 3H), 1.28 (s, 6H).

**Preparation of S-methyl 4-methyl-4-[methyl-[2-(2-naphthylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N,2-dimethyl-N-[2-(2-naphthylmethoxy)ethyl]but-3-yn-2-amine. Scale: 0.65 mmol. Purification by chromatography on silicagel (petroleum ether/EtOAc=85/15). Yield: 28%. Yellow oil.
1H NMR (300 MHz, DMSO) δ7.95 - 7.82 (m, 4H), 7.55 - 7.41 (m, 3H), 4.66 (s, 2H), 3.57 (t, J = 6.2Hz, 2H), 2.66 (t, J = 6.1 Hz, 2H), 2.38 (s, 3H), 2.28 (s, 3H), 1.37 (s, 6H).
ESI - HRMS : calc. for C₂₁H₂₆NO₂S 356.1683, found 356.1679 [M+H]+

### Example 16: S-methyl 4-methyl-4-[methyl-[2-[(2,6,6-trimethylcyclohexen-1-yl)methoxy]ethyl]amino]pent-2-ynethioate

**Preparation of N,2-dimethyl-N-[2-[(2,6,6-trimethylcyclohexen-1-yl)methoxy]ethyl]but-3-yn-2-amine:** The compound is obtained by using the same process as the one described for N-(2-allyloxyethyl)-N,2-dimethyl-but-3-yn-2-amine [example 2] using 1.015 eq of NaH and 1.01 eq. of 2-(Bromomethyl)-1,3,3-trimethyl-1-cyclohexene (prepared from β-Cyclocitral by known protocols (WO 2015048363)). Purification by chromatography on silicagel (petroleum ether/EtOAc=95/05). Yield: 67%. Pale yellow oil.
3.86 (s, 2H), 3.41 (t, J = 6.5Hz, 2H), 3.12 (s, 1H), 2.53 (t, J = 6.4Hz, 2H), 2.20 (s, 3H), 1.90 (t, J = 5.9Hz, 2H), 1.62 (s, 3H), 1.57 - 1.49 (m, 2H), 1.41 - 1.33 (s, 2H), 1.27 (s, 6H), 0.97 (s, 6H).

**Preparation of S S-methyl 4-methyl-4-[methyl-[2-[(2,6,6-trimethylcyclohexen-1-yl)methoxy]ethyl]amino]pent-2-ynethioate:** The compound is obtained by using the same process as the one described for S-methyl 4-[2-ethoxyethyl(methyl)amino]-4-methyl-pent-2-ynethioate (example 15) starting from N,2-dimethyl-N-[2-[(2,6,6-trimethylcyclohexen-1-yl)methoxy]ethyl]but-3-yn-2-amine. Purification by chromatography on silicagel (petroleum ether/EtOAc=90/10). Yield: 63%. Yellow oil.
H NMR (300 MHz, DMSO) δ 3.87 (s, 2H), 3.44 (t, J = 6.3 Hz, 2H), 2.57 (t, J = 6.3 Hz, 2H), 2.38 (s, 3H), 2.26 (s, 3H), 1.91 (t, J = 6.2 Hz, 2H), 1.62 (s, 3H), 1.57 - 1.49 (m, 2H), 1.36 (s, 6H), 1.39 - 1.34 (m, 2H), 0.97 (s, 6H).
ESI - HRMS calc for C20H34NO2S [M+H]+: 352.2305, found : 352.2289.

### Example 17: S-methyl 2,5,10,11,11-pentamethyl-6-oxo-7-oxa-2,5,10-triazatetradec-12-yne-14-thioate

**Preparation of compound 6:** A solution of p-nitrochloroformate (140 mg, 0.70 mmol, 1.5 eq) in dichloromethane (DCM) (2.5 mL) is added dropwise to a solution of compound 5 of Example 14 (100 mg; 0.46 mmol) with triethylamine (TEA) (1.5 eq) in DCM (1.5 mL) at 0°C. After 10 minutes at 0°C, the reaction mixture is heated to room temperature with stirring until complete conversion (verification by TLC, 1h). The mixture is then diluted in DCM (30 mL) then washed washed with brine (30 mL). The organic layer was dried over sodium sulfate, filtered and concentrated in vacuo. Purification by chromatography on silica gel (petroleum ether/ethyl acetate 100/70/30) to give compound 6 in the form of an amorphous solid (yield 82%).
1H NMR (300MHz, DMSO): δ (ppm) 8.35-8.30 (m, 2H), 7.59-7.53 (m, 2H), 4.30 (t, J = 5.7 Hz, 2H), 2.75 (t, J = 5.7 Hz , 2H), 2.39 (s, 3H), 2.30 (s, 3H), 1.39 (s, 6H).

**Preparation of S-methyl 2,5,10,11,11-pentamethyl-6-oxo-7-oxa-2,5,10-triazatetradec-12-yne-14-thioate:** To compound 6 of example 18 (200 mg, 0.53 mmol) in 3 mL of DCM (3 mL) is added TEA (1.2 eq) at room temperature. To the reaction mixture, a solution of le N, N, N'-triméthyléthylènediamine (1.2 eq) in dichloromethane (2.3 mL) is added at 0° C. The bright yellow reaction mixture is warmed to room temperature and stirred overnight. The reaction mixture, a bright yellow color, was stirred overnight at room temperature, diluted with dichloromethane (40 mL) then washed with brine (30 mL). The organic layer was dried over sodium sulfate, filtered and concentrated in vacuo. Purification by chromatography on silica gel (petroleum ether / ethyl acetate 75/25 to 20/80). Yield: 70%. Oil.
Purification by chromatography on silica gel DCM/MeOH (85/15). Yield: 34%. Yellow oil. 1H NMR (300 MHz, DMSO) δ 4.01 (t, J = 5.9Hz, 2H), 3.32-3.25 (m, 2H), 2.83 (large s, 3H), 2.62 (t, J = 5.8Hz, 2H), 2.6-2.5 (m partially hide by solvent peak, 2H), 2.39 (s, 3H), 2.27 (s,3H), 2.17 (large s, 6H), 1.36 (s, 6H).
ESI-HRMS calc for C16H30N3O3S [M+H]+: 344.1992, found : 344.1993.

### Example 18: S-methyl 4-(((1R,2R)-2-(benzyloxy)cyclopentyl)(methyl)amino)-4-methyl pent-2-ynethioate

**Preparation of (1R, 2R)-2-benzyloxy-N-(1,1-dimethylprop-2-ynyl)cyclopentanamine** : To a solution of commercially available (1R,2R)-2-(benzyloxy)cyclopentanamine (0.93 g, 4.86 mmol), 3-chloro-3-methylbut-1-yne (1.3eq) and triethylamine (1.3eq) in THF (20 mL) was added Cul (8 mol%) at room temperature. The mixture was left to stir overnight. The solvent was evaporated under reduced pressure and the crude was then diluted in aqueous saturated NaHCO₃ solution, extracted with ethyl acetate. Combined organic layers were washed with 2% NH₄OH aqueous solution then brine, dried over Na₂SO₄ and the solvent evaporated under reduced pressure. (1R, 2R)-2-benzyloxy-N-(1,1-dimethylprop-2-ynyl)cyclopentanamine was obtained as a brown oil. Yield : 99%.
¹H NMR (300 MHz, DMSO) δ 7.37 - 7.16 (m, 5H), 4.56 - 4.36 (m, 2H), 3.72 - 3.58 (m, 1H), 3.28 - 3.19 (m, 1H), 3.08 (s, 1H), 2.00 - 1.88 (m, 1H), 1.86 - 1.67 (m, 2H), 1.65 - 1.49 (m, 3H), 1.40 - 1.28 (m, 1H), 1.25 (s, 3H), 1.25 (s, 3H).

**Preparation of (1R,2R)-2-(benzyloxy)-N-methyl-N-(2-methylbut-3-yn-2-yl)cyclopentanamine** : To (1R,2R)-2-(benzyloxy)-N-(2-methylbut-3-yn-2-yl)cyclopentanamine (0.25 g, 0.97 mmol) were added 5 eq of formic acid and 1.5 eq of formaldehyde (37% in water). The mixture was refluxed overnight then 2N HCI was added until pH 1 was reached and washed with ether. The aqueous layer was basified with 1N NaOH, and extracted with DCM. The organic layer was washed with brine, dried over Na₂SO₄ and the solvents evaporated under reduced pressure. The crude is then purified by chromatography on silica gel (petroleum ether/EtOAc = 80/20), giving a yellow oil. Yield : 68%.
¹H NMR (300 MHz, DMSO) δ 7.43 - 7.17 (m, 5H), 4.52(m, 2H), 3.94 - 3.78 (m, 1H), 3.62 - 3.49 (m, 1H), 3.11 (s, 1H), 2.20 (s, 3H), 1.78 - 1.45 (m, 6H), 1.37 (s, 3H), 1.34 (s, 3H). ESI - LRMS: 272.1 [M+H]⁺.

**Preparation of S-methyl 4-(((1R,2R)-2-(benzyloxy)cyclopentyl)(methyl)amino)-4-methylpent-2-ynethioate** : The compound was obtained by using the same process as the one described for S-methyl 4-[2-ethoxyethyl(methyl)amino]-4-methyl-pent-2-ynethioate [example 1] starting from (1R,2R)-2-(benzyloxy)-N-methyl-N-(2-methylbut-3-yn-2-yl)cyclopentanamine. Purification by chromatography on silica gel (petroleum ether/EtOAc= 80/20 then DCM = 100). Scale 0.64 mmol. Yield : 47%. Yellow oil.
¹H NMR (300 MHz, DMSO) δ 7.35 - 7.20 (m, 5H), 4.51 (s, 2H), 3.92 - 3.80 (m, 1H), 3.59 - 3.45 (m, 1H), 2.36 (s, 3H), 2.24 (s, 3H), 1.78 - 1.49 (m, 6H), 1.45 (s, 3H), 1.42 (s, 3H). ESI - HRMS calc for C₂₀H₂₈NO₂S [M+H]+: 346.1835, found: 346.1824.

### Example 19: (S)-S-methyl 4-(2-((benzyloxy)methyl)pyrrolidin-1-yl)-4-methylpent-2-ynethioate:

**Preparation of (S)-2-((benzyloxy)methyl)-1-(2-methylbut-3-yn-2-yl)pyrrolidine:** The compound was obtained by using the same process as the one described for N-(2-allyloxyethyl)-N,2-dimethyl-but-3-yn-2-amine [example 2] starting from (S)-(1-(2-methylbut-3-yn-2-yl)pyrrolidin-2-yl)methanol 1.015 eq of NaH and 1.01 eq. of benzyl bromide. Purification by chromatography on silicagel (dichloromethane/methanol = 95/5). Scale 3.0 mmol. Yield : 60%. Orange oil.
¹H NMR (300 MHz, DMSO) δ 7.41 - 7.19 (m, 5H), 4.46 (s, 2H), 3.24 (dd, J = 8.3, 2.7 Hz, 1H), 3.14 (dd, J = 7.6, 3.2 Hz, 1H), 3.11 - 3.02 (m, 2H), 2.86 (dd, J = 8.4, 6.0 Hz, 1H), 2.65 - 2.54 (m, 1H), 1.81 - 1.51 (m, 4H), 1.25 (s, 3H), 1.23 (s, 3H).
ESI-LRMS: 258.1 [M+H]+.

**Preparation of (S)-S-methyl 4-(2-((benzyloxy)methyl)pyrrolidin-1-yl)-4-methylpent-2-ynethioate:** The compound was obtained by using the same process as the one described for S-methyl 4-[2-ethoxyethyl(methyl)amino]-4-methyl-pent-2-ynethioate [example 1] starting from (S)-2-((benzyloxy)methyl)-1-(2-methylbut-3-yn-2-yl)pyrrolidine. Purification by chromatography on silicagel (petroleum ether/EtOAc=80/20). Scale 1.2 mmol. Yield : 50%. Orange oil.
¹H NMR (300 MHz, DMSO) δ 7.46 - 7.18 (m, 5H), 4.47 (s, 2H), 3.29 - 3.23 (m, 1H), 3.17 - 3.03 (m, 2H), 2.94 (dd, J = 8.5, 5.7 Hz, 1H), 2.62 - 2.53 (m, 1H), 2.38 (s, 3H), 1.85 - 1.58 (m, 4H), 1.34 (s, 3H), 1.32 (s, 3H).

### Example 20: (R)-S-methyl 4-(3-(benzyloxy)pyrrolidin-1-yl)-4-methylpent-2-ynethioate:

**Preparation of (R)-3-(benzyloxy)-1-(2-methylbut-3-yn-2-yl)pyrrolidine** : The compound was obtained by using the same process as the one described for N-(2-allyloxyethyl)-N,2-dimethyl-but-3-yn-2-amine [example 2] starting from (R)-1-(2-methylbut-3-yn-2-yl)pyrrolidin-3-ol using 1.01 eq of NaH and 1.01 eq. of benzyl bromide. Purification by chromatography on silicagel (dichloromethane/methanol = 98/2). Scale 3.3 mmol. Yield : 69%. Orange oil.
¹H NMR (300 MHz, DMSO) δ 7.40 - 7.17 (m, 5H), 4.42 (s, 2H), 4.07 (tt, J = 7.3, 3.7Hz, 1H) 3.13 (s, 1H), 2.86 (dd, J = 9.6, 6.7 Hz, 1H), 2.75 - 2.65 (m, 2H), 2.60 - 2.53 (m, 1H), 2.06 - 1.95 (m, 1H), 1.75 - 1.65 (m, 1H), 1.29 (s, 6H).
ESI - LRMS 244.1 [M+H]+.

**Preparation of (R)-S-methyl 4-(3-(benzyloxy)pyrrolidin-1-yl)-4-methylpent-2-ynethioate:** The compound was obtained by using the same process as the one described for S-methyl 4-[2-ethoxyethyl(methyl)amino]-4-methyl-pent-2-ynethioate [example 1] starting from (R)-3-(benzyloxy)-1-(2-methylbut-3-yn-2-yl)pyrrolidine. Purification by chromatography on silicagel (dichloromethane/methanol=98/2). Scale 1.2 mmol. Yield : 69%. Orange oil.
¹H NMR (300 MHz, DMSO) δ 7.32 (m, 5H), 4.44 (s, 2H), 4.09 (dq, J = 10.2, 3.5 Hz, 1H), 2.97 - 2.83 (m, 1H), 2.80 - 2.65 (m, 2H), 2.65 - 2.55 (m, 1H), 2.37 (s, 3H), 2.11 - 1.90 (m, 1H), 1.83 - 1.67 (m, 1H), 1.37 (s, 6H).
ESI - HRMS calc for C₁₈H₂₄NO₂S [M+H]+: 318.1522, found: 318.1518.

### Example 21: Preparation of S-methyl 4-dimethylamino-4-methyl-pent-2-ynethioate

To a solution of 0.855g of 3-dimethylamino-3-methyl-but-1-yne [HENNION, G.F., NELSON, K.W. (1957) J. Am. Chem. Soc., 79, 2142-2144] (2.80 mmol) in 14 ml of tetrahydrofuran, 4.07 ml (9.24 mmol) of a solution of 2.27 M denbutyllithium in hexane are added in 5 minutes at -70°C. After 5 minutes, the reaction medium is brought to 0°C and stirred for a further 30 minutes at this temperature. After returning to -70°C, 2 ml of carbon oxysulphide previously condensed are cannulated and the mixture is stirred for 30 minutes at -70°C. The reaction medium is then brought to 0°C over 30 minutes and then 0.575 ml (9.24 mmol) of methyl iodide is added and stirring is continued for 2 hours at 0°C. Diluted in 250 ml of ether, washed with saturated sodium chloride solution (3 × 30 ml), dried over sodium sulfate. After evaporation under vacuum and purification by chromatography on silica gel (elution with a petroleum ether/ethyl acetate mixture = 70/30), 1.16 g of the compound were isolated in the form of a colorless oil (yield: 81%).
¹H NMR (300 MHz, CDCl3): v = 1.42 (s, 6H, (CH3) 2C), 2.31 (s, 6H, (CH3) 2N), 2.39 (s, 3H, CH3S).

### Example 22: Preparation of S-methyl 4-methyl-4-morpholin-4-yl-pent-2-ynethioate

The preparation is identical to that described in Example 21 using 3-di-n-propylamino-3-methyl-but-1-yne [HENNION, G.F., HANZEL, R.F., J. Am. Chem. Soc., 82, 4908-4912, (1960)] in place of 3-methyl-3-morpholin-4-yl-but-1-yne. Quantity: 1.5 mmol, purification by chromatography on silica gel (eluent petroleum ethe /ethyl acetate = 60/40), yield: 77%. Solid white.
¹H NMR (300 MHz, CDCl3): v = 1.43 (s, 6H, (CH3) 2C), 2.40 (s, 3H, CH3S), 2.65 (m, 4H, CH2O), 3.97 (m, 4H, CH2N).

### Example 23: Preparation of S methyl 4-methyl-4- [methyl (octyl) amino] pent-2-ynethioate

To N-methyl-N-(2-methylbu-3-yn-2-yl) hetan-1-amine (511 mg, 2.62 mmol) in solution in anhydrous THF (5 mL) is added a solution of nBuLi 2.28 M in hexane (1.26 mL, 1.1 eq) dropwise at -70°C. The reaction medium is brought to 0°C and CO₂ gas is introduced into the solution by bubbling through a needle for 30 min. The bubbling is continued for a further 5-10 min while the solution rises to room temperature. After returning to 0°C, isobutyl chloroformate (1.1 eq) is added dropwise. Stirring is continued during 15 min before adding MeSNa (1.2 eq) in one portion. The reaction medium is brought to room temperature and stirred for 15 min before hydrolysis with water. The treatment consists of extraction with ethyl acetate (x3) followed by washing the combined organic phases with a saturated aqueous solution of sodium chloride. After drying over Na₂SO₄ and concentrating on a rotary evaporator, the reaction crude is purified by chromatography on silica gel (petroleum ether/EtOAc = 95/5) to give the thiolester (445 mg). Yield: 63%. Solid white.
1H NMR (300 MHz, DMSO) δ 2.38 (s, 3H), 2.37 (t, J = 7.0 Hz, 2H), 2.18 (s, 3H), 1.35 (s, 6H), 1.42 - 1.20 (m, 10H), 0.86 (t, J = 6.7Hz, 3H). 13C NMR (75 MHz, CDCl3) δ 176.38 (C), 96.50 (C), 81.03 (C), 54.98 (C), 52.39 (CH2), 36.39, 31.85 (CH2), 29.25 (CH2), 28.79 (CH2), 27.81 (2CH3), 27.41 (CH2), 22.62 (CH2), 14.09 (CH3), 12.41 (CH3). ESI-LRMS 270.2 [M + H] +.

### Part 2- Use of the compounds of formula (I)

### MATERIALS AND METHODS

### Flow cytometry immunophenotyping

Immunocompetent female C57BI6 mice were administered with DIMATE (15 mg/kg), anti-PDL1 (12.5mg/kg) or PBS control, three times per week (i.v.) for a total period of 21 days. Blood samples were obtained at end point and were collected in Heparin.

In a different setting, immunocompetent mice models were implanted subcutaneously with tumor cells (see description below under "Animal models of cancer immunotherapy"). When tumors reached 50-100 mm³ volume, mice were administered with DIMATE (15 mg/kg), anti-PDL1 (12.5mg/kg) or PBS control, three times per week (i.v.) for a total period of 21 days. Tumors were obtained at end point, enzymatically digested with hyaluronidase and collagenase.

Cells were homogenized using GentleMACS cell homogenization before immunostaining. Lymphoid panels (T effector and NK cells) and myeloid panels (Granulocytes, neutrophiles, eosinophiles, basophiles, macrophages type M2, intermediary macrophages and monocytes) of conjugated antibodies were from Beckman Coulter as described in Table 2.

**Table 2: Immunophenotyping flow cytometry panels used to analyze the different immune cell populations.**

| Lymphoid panel | |
|---|---|
| Populations | Markers |
| T effector | CD45 + CD3 + CD8 + CD107 + GranzymeB+ |
| NK | CD45 + CD3 - CD11b + CD49b+ |

| Myeloid panel | |
|---|---|
| Populations | Markers |
| Granulo, neutro, eosino, baso | CD45 + CD3 - CD11c - CD64 + CD11b + CD68 +Ly6G- |
| M2 | CD45 + CD3 - CD11c - CD64 + CD11b + CD68 +Ly6G- CMHII++ |
| M intermediaire | CD45 + CD3 - CD11c - CD64 + CD11b + CD68 +Ly6G-Ly6C -/+ CMHII+ |
| Monocytes | CD45 + CD3 - CD11c - CD64 + CD11b + CD68 +Ly6G-Ly6C ++ CMHII- |

PerfixNC kit was purchased from Beckman Coulter.

Whole blood (100 µl) sampled with anticoagulant was mixed with 10 µl of Perfix-NC R1 buffer, vortexed immediately for 2-3 s and incubated for 15 min at room temperature in the dark. About 600 µl of Perfix-NC R2 buffer were added, 355 µl were transferred in a Duraclone tube and the corresponding antibodies were added. After vortexing, tubes were incubated for 60 min at room temperature in the dark. PBS 1 × (3 ml) was added to the tubes, incubated for 5 min at room temperature in the dark before centrifugation for 6 min at 250g. The supernatant was removed to leave the pellet dried and the cells were resuspended in 3 ml of 1 × Perfix-NC R3 buffer prior to another 6-min centrifugation at 250g. The pellet was dried and resuspended in 300 µl of 1× R3 buffer. Tubes were protected from light and stored at 40°C until the acquisition on a flow cytometer within the next 24 h.

Flow cytometry data were analysed with Kaluza 1.3 software (Beckman Coulter). Statistical analyses were performed using Prism 6.0 (GraphPad) software. T test Mann-Whitney was used to compare matched pairs. P < 0.05 was considered as significant. **RT-PCR analysis**HL-60 AML cell line was used to perform the transcriptome analysis. This cell line was purchased from ATCC. HL-60 were cultured in RPMI 1640 Medium, GlutaMAX^{™} Supplement (Gibco^{™}), and 10% Foetal Bovine Serum and maintained at 37°C, 5% CO₂ in a humid incubator. There were 4 replicates for each condition: not treated cells and cells treated with 10µM of DIMATE for 6h in 6-well plate at a density of 500 000 cells/well in a culture media total volume of 3mL total of media. After incubation, cells were collected, washed 3 times with PBS and the pellet processed for RNA extraction.

Total RNA was extracted using RNeasy Mini kit (Qiagen, France) according to manufacturer's instructions including DNase treatment. RNAs were quantified with NanoDrop 1000 spectrophotometer (Nano Drop Technologies, San Diego, CA,USA). Optical density was measured at 260 and 280 nm and the ratio 260/280 (> 1.8) was retained as index of good purity. The quality of the extracted RNA was verified utilizing Agilent 2100 Bioanalyzer ^{™} (Agilent Technologies, Santa Clara, CA,USA). A score on a scale of 0 to 10 was automatically attributed to each sample and corresponded to RNA Integrity Number (RIN). Samples had a RIN value of 9-10.

Subsequently, one hundred nanogram of total RNA was labeled using One-Color Microarray-Based Gene Expression Analysis: Low Input Quick Amp (LIQA) labeling protocol. 0.6 µg of the purified Cy3 labeled cRNA were hybridized for 17h at 65°C, at 60 rpm, using the SurePrint G3 human GE 8x60K V2 chips (Agilent Technologies, Santa Clara, California). Microarrays were composed of 62 928 features. Probes synthesized on chips had a size of 60 nucleotides. Microarrays were washed using Gene Expression Wash Buffer Kit (Agilent Technologies) and scanned through standard Agilent protocol. Data were processed using Feature Extraction software.

The library AgiND was implemented in R software to analyze and visualize data. AgiND was developed on Bioconductor library model and was used to diagnose data quality and data microarrays normalization. Quantile method was used to normalize data to homogenize the distribution of the microarray intensity. A filter was applied on row data to delete controls, then a second filter was applied to delete genes which were expressed under the background in at least 100% of samples in each group.

To identify the genes differentially expressed between: Not treated HL-60 and HL-60 treated with DIMATE a tree class Significance Analysis of Microarray (SAM) test was performed on the normalized and filtered data. A multiple test correction was performed a false discovery rate (FDR) of 5% were fixed. A supervised hierarchical clustering was applied on adjusted gene expression median adjusted data to group genes and samples, according to their expression using « TMeV» (Tigr MultiExperiment Viewer) MeV: MultiExperiment Viewer | Part of the TM4 Microarray Software Suite (2). Pearson correlation was used, and clusters were grouped on basis of average linkage method. Differentially expressed genes of interest (custom list of genes) was visualized in heatmap.

### Capillary electrophoresis (WES)

For protein expression analysis, cells were lysed in RIPA buffer (50 mM Tris-HCI pH7, 1% NP-40, 0.5% Na-deoxycholate, 0.1% SDS, 150mM NaCl and 2mM EDTA) containing 1/200 protease inhibitor (539134-1ML, Millipore Sigma) and 1/100 phosphatase inhibitors (524625-1SET, Millipore Sigma). Proteins were quantified using a Lowry method with a kit Pierce^{™} BCA^{®} Protein Assay (23225, Thermo Fisher Scientific).

Total protein extracts were separated on a WES system (ProteinSimple) according to the manufacturer's instructions using a 12-230 kDa separation module (ProteinSimple, SM-W004) and the Assay Module Anti-rabbit HRP Detection Module (ProteinSimple, DM-001). Samples were diluted at 0.5 mg/mL in Sample Buffer 0.1X (10x Buffer provided in the separation module) then mixed with Fluorescent Master Mix, vortexed and heated 5min at 95°C then kept on ice until use. The samples, and the blocking reagent (antibody diluent), primary antibodies (in antibody diluent), HRP-conjugated secondary antibodies and chemiluminescent substrate provided in the detection module, were pipetted into the plate (also provided in Separation Module).

Instrument default settings were used: stacking and separation at 375 V for 25 min; blocking reagent for 5 min, primary and secondary antibody both for 30 min; Luminol/peroxide chemiluminescence detection for approximatively 15 min (exposures of 1-2-4-8-16-32-64-128-512s). The resulting graphs were analysed and visualized as lanes. Primary antibodies for XIAP and clAPs proteins were from Cell Signaling Technology.

### NK lytic activity

NK cell specific lytic activity was evaluated in the absence or presence of DIMATE (10µM) using NK cells obtained from healthy human PBMCs from freshly collected cytapheresis and K562, a human erythroleukemic tumour cell line that do not express class I MHC molecules, as the target population.

Two fluorescent dyes were used to discriminate between effector and target cells and between live and dead target cells. The green-fluorescent dye 3,3'- Diocta-decyloxacarbo-cyanine perchlorate (DIOC, Sigma/Merck D4292-20MG) was used to label the plasma membranes of K562 following manufacturer instructions. Briefly, 1.106 K562cells were resuspended in RPMI (phenol free) and 5µl of DIOC (1mM) were added. Cells were incubated at 37°C 5% CO₂ for 10 minutes before washing away the excess of DIOC with media using three centrifugation cycles of 250g, 5 min each.

Healthy PBMCs were pre-treated with DIMATE (10µM) for 6 hours. Then, the drug was washed away and PBMCs were incubated with K562 target cells for 21 hours. The second dye, propidium iodide (PI), a membrane impermeable, red fluorescent dye, was added at the end of the assay to counter-stain the targets cells with a compromised cell membrane as a result of the NK cells lytic activity.

Fluorescence was monitored by flow cytometry before and after target-effector contact. The test was performed on a total of 10 healthy donors. Percent of cell lysis was calculated as follows: [Cells positive for both DIOC and PI/Total DIOC labelled cells] * 100) - spontaneous lysis.

### Lipidic nanoparticle formulation of compounds

The aminothiolester compounds (e.g. DIMATE) were formulated as lipidic nanoparticles consisting of 2.5% of Lipoïd S100 (Lipoid gmbh, Ludwigshafen, Germany), 20% Labrafac Lipophile WL1349 (Gattefosse, France), 17% Kolliphor HS15 (Sigma), 3% Sodium chloride (NaCl, (AppliChem), 60% of H20 and 12.2 mg/mL of an aminothiolester compound (e.g. DIMATE). The procedure was as follows: DIMATE (or another aminothiolester compound) was mixed with Labrafac and left steering until completely dissolved. In a separate falcon, Kolliphor, Lipoïd, NaCl and the water were mixed. This last solution (acqueous phase) was transferred into the oily phase (DIMATE plus Labrafac) under steering, placed into a silicon oil bath (Silicon oil -50°C - +200°C, Sigma Aldrich) and heated up to 85°C under vigorous steering. Repeated cooling and heating steps were performed three times. At the end of the last heating step, the mixture was transferred into an ice bath and cooled down to 70°C. As soon as the formulation reached 70°C, cold water (4°C) was added to quench and the formulation was left in further agitation for 5 minutes.

### Animal models of cancer immunotherapy

To test the *in vivo* efficacy of LNP-DIMATE alone or in combination with the immune-checkpoint inhibitor anti-PDL-1, three different animal models were generated in immunocompetent female C57BI6 mice (4-weeks-old). 2x 10⁶ CT26 mice cells for colon cancer model, LLC2 mice cells for lung cancer model or Tyr-NRAS mice cells for melanoma model (from Antineo, Lyon, France) were implanted subcutaneously in the animal flanks. When tumors reached 50-100 mm³ volume, mice were randomly divided into the isotype control group, empty lipid nanoparticles (LNP) group, anti-PD-L1 group, LNP encapsulated DIMATE (LNP-DIMATE) group, LNP-DIMATE plus Isotype control group and LNP-DIMATE plus anti-PDL1. Each group consisted of 10 animals. Anti-PDL1 and the isotype control were administered at 12.5 mg/kg. LNP and LNP-DIMATE were administered at 15mg/kg. All the treatments were performed three times per week (i.v.) for a total period of 21 days. Tumor volume measured by a caliper was used to monitor response to treatment.

### RESULTS

Results are illustrated by Figures 2 to 6.

In particular, it shows that:
- Compounds of formula (I) increased ROS, promoted granulopoiesis, increased neutrophils mobilization and expression of CD11b- a component that is essential for neutrophil recruitment and control of infection (Figure 2). This characteristic is useful to promote neutrophil recovery in the treatment of patients with low neutrophil count, especially useful in managing patients with drug-induced neutropenia including chemotherapeutic agents such as alkylating agents, anthracyclines, camptothecins, mitomycin C, taxanes, vinblastine, hydroxyurea, among others and non-chemotherapeutic drugs that induce late-onset neutropenia which have the potential to be a long-lasting complication such as rituximab, carbimazole, clozapine, dapsone, dipyrone, methimazole, oxacillin, vancomycin, penicillin G, procainamide, propylthiouracil, sulfasalazine, and ticlopidine and commonly used drugs that cause neutropenia at lower rates such as angiotensin converting enzyme (ACE) inhibitors, H2 blockers, non-steroidal anti-inflammatory drugs (NSAIDs), and antiarrhythmic drugs, among others;
- Compounds of formula (I) induced mobilization of monocyte-associated macrophages, M2-type macrophage activation, well known for their role in pathogen defense, but also in the clearing apoptotic cells, promoting wound healing and mitigating exacerbated inflammatory responses (Figure 3). Accordingly, compounds of formula (I) also induced anti-inflammatory molecules such as the M2 markers CHI3L1 and AXL while exhibiting anti-inflammatory functions via downregulation of TRAF6, inhibition of the NF-kB signaling pathway and decreased gene expression of NFkB-dependent pro-inflammatory cytokines such as CCL4, IL-1b and TNFα. Importantly, compounds of formula (I) induced VEGFA and TGFα, which are implicated in cell proliferation, cell differentiation, wound healing, and promotion of angiogenesis (Figure 4). These data show that the compounds of formula (I) can be used in the context of tissue repair and regeneration, to mitigate the uncontrolled amplitude and duration of inflammatory processes, known to prevent regeneration, or for instance to cause chronic inflammatory environment, such as in rheumatoid arthritis;

Compounds of formula (I) induced T effector cells, and NK cells recruitment in the intratumor environment- which are known to promote immune antitumor response (Figure 5A). Consistently with these findings, compounds of formula (I) have a synergistic effect with immune-checkpoint blockade, showing that their combination with immune-checkpoint inhibitors (ICI) like anti-PDL-1 is a promising strategy to enhance their anti-tumor effect (Figure 5B), and compounds of formula (I) does not affect the viability nor the lytic activity of human NK cells *in vitro,* and it induces degradation of XIAP and clAP proteins. The blockade of these intrinsic factors using IAP antagonists, have been demonstrated to disrupt the TRAF-cIAP E3 complex, and provide promising results in animal models of cancer immunotherapy (Figure 6).

## Claims

1. At least one compound of formula (I): 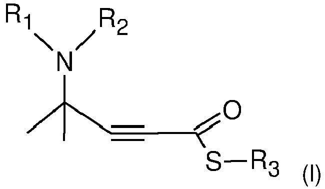 wherein:
- R₁ and R₂, identical or different, are chosen among a C₁-C₁₀ alkyl group, a phenyl, a benzyl, CHR₅CHR₆OR₄ and (CHR₅)ᵥOR₄, or R₁ and R₂ together with the nitrogen atom to which they are linked form an heterocycle, in particular a piperidine or a morpholine;
said phenyl and benzyl being optionally substituted by one or more substituents chosen from: linear or branched (C₁-C₇)alkyl, halogen, NO₂ and CONH₂ ;
- R₃ is chosen from linear or branched (C₁-C₇)alkyl,
- R₄ is chosen from: H, linear or branched (C₂-C₇)alkyl, linear or branched (C₂-C7)alkenyl, -CONR₇R₈, aryl, heteroaryl, (C₂-C₇)cycloalkyl, linear or branched -(C₁-C₇)alkyl-aryl and linear or branched -(C₁-C₇)alkyl-heteroaryl;
said aryl, (C₂-C₇)cycloalkyl, and heteroaryl being optionally substituted by one or more substituents chosen from: halogen, linear or branched (C₁-C₇)alkyl optionally substituted by one or more halogen atom, linear or branched (C₁-C₇)alkoxy optionally substituted by one or more halogen atom, -COOH, aryl, -NRR', -NO₂, or said aryl and heteroaryl being optionally fused to form an heterocycloalkyl;
- R₅ and R₆ identical or different are independently chosen from:
• H and linear or branched (C₁-C₇)alkyl, or
• R₅ and R₆ are linked together to form with the carbon atoms to which they are attached a cycloalkyl, aryl or heteroaryl, or
• R₅ is H and R₁ and R₆ are linked together to form with the nitrogen atom linked to R₁ an heterocycloalkyl or heteroaryl, or
• R₆ is H and R₁ and R₅ are linked together to R₁ to form with the nitrogen atom linked to R₁ an heterocycloalkyl;
- v is chosen from 2 to 4;
- R₇ is -(C₁-C₃)alkyl;
- R₈ is -(C₁-C₃)alkylNRR';
- R and R' identical or different, are independently chosen from H and linear or branched (C₁-C₇)alkyl,
or its pharmaceutically acceptable salts or optical isomers, racemates, diastereoisomers, enantiomers or tautomers;
for use for modulating the immune response in a subject.

2. At least one compound for use according to claim 1, wherein R₃ is linear or branched (C₁-C₇)alkyl, preferably methyl, R₁ is linear or branched (C₁-C₇)alkyl, preferably methyl, R₂ is a C₁-C₁₀ alkyl group, preferably a methyl, CHR₅CHR₆OR₄, (CHR₅)ᵥOR₄ or R₁ and R₂ together with the nitrogen atom to which they are linked form an heterocycle, in particular a morpholine, and R₅ and _{R6} are H.

3. At least one compound for use according to claim 1 or 2, wherein R₅ and R₆ are H and R₄ is chosen from (C₂-C₇) cycloalkyl, linear or branched -(C₁-C₇)alkyl-heteroaryl, or benzyl; preferably benzyl; said (C₂-C₇) cycloalkyl being substituted by one or more substituents chosen from: linear or branched (C₁-C₇)alkyl, said benzyl being optionally substituted by one or more substituents chosen from: linear or branched (C₁-C₇)alkyl optionally substituted by one or more halogen atom, linear or branched (C₁-C₇)alkoxy optionally substituted by one or more halogen atom, halogen.

4. At least one compound for use according to any of the preceding claims, wherein said at least compound of formula (I) is chosen from:
- S-methyl 4-[2-ethoxyethyl(methyl)amino]-4-methyl-pent-2-ynethioate;
- S-methyl 4-[2-allyloxyethyl(methyl)amino]-4-methyl-pent-2-ynethioate;
- S-methyl 4-[2-benzyloxyethyl(methyl)amino]-4-methyl-pent-2-ynethioate;
- S-methyl 4-methyl-4-[methyl-[2-(m-tolylmethoxy)ethyl]amino]pent-2-ynethioate;
- S-methyl 4-[2-[(3,4-dimethylphenyl)methoxy]ethyl-methyl-amino]-4-methyl-pent-2-ynethioate;
- S-methyl 4-[2-[(4-methoxyphenyl)methoxy]ethyl-methyl-amino]-4-methyl-pent-2-ynethioate;
- S-methyl 4-[2-[(3,4-dimethoxyphenyl)methoxy]ethyl-methyl-amino]-4-methyl-pent-2-ynethioate;
- S-methyl 4-[2-[(3-chlorophenyl)methoxy]ethyl-methyl-amino]-4-methyl-pent-2-ynethioate;
- S-methyl 4-[2-[(3-fluorophenyl)methoxy]ethyl-methyl-amino]-4-methyl-pent-2-ynethioate;
- S-methyl 4-methyl-4-[methyl-[2-(2-pyridylmethoxy)ethyl]amino]pent-2-ynethioate;
- S-methyl 4-methyl-4-[methyl-[2-(3-pyridylmethoxy)ethyl]amino]pent-2-ynethioate;
- S-methyl 4-methyl-4-[methyl-[2-(4-pyridylmethoxy)ethyl]amino]pent-2-ynethioate;
- S-methyl 4-((4-(benzyloxy)butyl)(methyl)amino)-4-methylpent-2-ynethioate;
- S-methyl 4-((2-hydroxyethyl)(methyl)amino)-4-methylpent-2-ynethioate;
- S-methyl 4-methyl-4-[methyl-[2-(2-naphthylmethoxy)ethyl]amino]pent-2-ynethioate;
- S-methyl 4-methyl-4-[methyl-[2-[(2,6,6-trimethylcyclohexen-1-yl)methoxy]ethyl]amino]pent-2-ynethioate;
- S-methyl 2,5,10,11,11-pentamethyl-6-oxo-7-oxa-2,5,10-triazatetradec-12-yne-14-thioate;
- S-methyl 4-methyl-4-[methyl(2-phenoxycyclopentyl)amino]pent-2-ynethioate;
- (S)-S-methyl 4-(2-((benzyloxy)methyl)pyrrolidin-1-yl)-4-methylpent-2-ynethioate;
- S-methyl 4-[3(benzyloxy)-1pyrrolidinyl])-4-methylpent-2-ynethioate;
- S-methyl 1-4-Dimethylamino-4-methyl-pent-2-ynethioate ;
- S-methyl 5- 4-Methyl-4-morpholin-4-yl-pent-2-ynethioate;
- S methyl 4-methyl-4- [methyl (octyl) amino] pent-2-ynethioate;
or its pharmaceutically acceptable salts or optical isomers, racemates, diastereoisomers, enantiomers or tautomers.

5. At least one compound for use according to any one of the preceding claims, wherein said at least one compound of formula (I) is comprised in a lipidic nanocapsule.

6. At least one compound for use according to claim 5, wherein said lipidic nanocapsule comprises:
- an oily core comprising between 25 and 90% by weight of medium chain triglycerides, preferably between 60 and 80%, relative to the total weight of the nanocapsule, and said at least one compound of formula (I); and
- a shell surrounding the oily core, comprising between 3 and 25% by weight relative to the total weight of the nanocapsule of at least one lipid surfactant, and at least one hydrophilic surfactant; and
• in which the ratio by weight relative to the total weight of the nanocapsule between the medium chain triglycerides and said at least one compound of formula (I) is of at least 4.

7. At least one compound for use according to any one of the preceding claims, wherein said at least one compound of formula (I) or said lipidic nanocapsule is comprised in a pharmaceutical composition.

8. At least one compound for use according to any one of the preceding claims for stimulating the immune response in a subject.

9. At least one compound for use according to any one of the preceding claims, for treating and/or preventing infectious pathologies, in particular via stimulation and promotion of maturation and/or activation of neutrophils.

10. At least one compound for use according to any one of the claims 1 to 8, for treating and/or preventing wound healing and/or tissue repair; autoimmune pathological conditions and/or metabolic diseases, in particular that cause inflammation.

11. At least one compound for use according to any one of the preceding claims, wherein said at least one compound of formula (I) is combined with an immune checkpoint inhibitor.

12. A pharmaceutical composition comprising at least one compound of formula (I) as defined in any of one claims 1 to 6, and an immune checkpoint inhibitor.

13. Products comprising at least one compound of formula (I) as defined in any one of claims 1 to 6and an immune checkpoint inhibitor as a combined preparation for simultaneous use, separate or spread over time as a medicament.

14. A pharmaceutical composition according to claim 12 or products according to claim 13 for use in the prevention and/or treatment of cancer.

15. A pharmaceutical composition or products for use according to claim 14, wherein said cancer is chosen from melanoma, breast cancer, lung cancer, prostate cancer, pancreatic cancer, colon cancer, acute myeloid leukemia, hepatocellular carcinoma, squamous cell head & neck cancer, renal cell carcinoma, cervical carcinoma, merkel cell carcinoma, PMBCL, classical Hodgkin lymphoma, gastric and GEJ carcinoma.

16. At least one compound for use according to claim 11, a pharmaceutical composition according to claim 12, products according to claim 13 or a pharmaceutical composition or products for use according to claim 14 or 15, wherein said immune checkpoint inhibitor is chosen from PD-1, PD-L1, PD-L2, CTLA-4, TIGIT, TIM-3, LAG-3 CD160 and 2B4 compounds, in particular from PD1/PDL1 compounds.
